# EUROPÄISCHE PATENTSCHRIFT

(11) **EP 1 709 043 B1**
(45) Veröffentlichungstag und Bekanntmachung des Hinweises auf die Patenterteilung: **03.12.2008**
(21) Anmeldenummer: 04798090.9
(22) Anmeldetag: 26.11.2004
(51) Int. Cl.: C07D 471/04

(54) **PYRROLOPYRIDIN-SUBSTITUTIERTE BENZOL-DERIVATE ZUR BEHANDLUNG KARDIOVASKULÄRER ERKRANKUNGEN**
PYRROLOPYRIDINE-SUBSTITUTED BENZOL DERIVATIVES FOR TREATING CARDIOVASCULAR DISEASES
DERIVES DU BENZOL SUBSTITUES PAR LA PYRROLOPYRIDINE, POUR LE TRAITEMENT DE MALADIES CARDIOVASCULAIRES

(30) Priorität: 09.12.2003 DE 10357510
(43) Veröffentlichungstag der Anmeldung: 11.10.2006
(73) Patentinhaber: Bayer HealthCare AG, 51368 Leverkusen (DE)
(72) Erfinder: BENNABI, Samir, 69300 Caluire et Cuire (FR); HECKROTH, Heike, 51519 Odenthal (DE); SCHIROK, Hartmut, 42287 Wuppertal (DE); MITTENDORF, Joachim, 42113 Wuppertal (DE); KAST, Raimund, 42349 Wuppertal (DE); STASCH, Johannes-Peter, 42651 Solingen (DE); GNOTH, Mark Jean, 40822 Mettmann (DE); MÜNTER, Klaus, 42489 Wülfrath (DE); LANG, Dieter, 42553 Velbert (DE); PEREZ, Santiago Figueroa, 51373 Leverkusen (DE); BAUSER, Marcus, 42115 Wuppertal (DE); FEURER, Achim, 69259 Wilhelmsfeld (DE); EHMKE, Heimo, 20251 Hamburg (DE)
(86) Internationale Anmeldenummer: PCT/EP2004/013430
(87) Internationale Veröffentlichungsnummer: WO 2005/058891

(56) Entgegenhaltungen:
- WO-A-02/22856
- WO-A-03/062227
- WO-A-03/082808
- FUKATA YUKO ET AL: "Rho-Rho-kinase pathway in smooth muscle contraction and cytoskeletal reorganization of non-muscle cells" TRENDS IN PHARMACOLOGICAL SCIENCES, ELSEVIER, AMSTERDAM, NL, Bd. 22, Nr. 1, Januar 2001 (2001-01), Seiten 32-39, XP002267445 ISSN: 0165-6147

## Beschreibung

Die Erfindung betrifft heteroarylsubstituierte Benzole, ein Verfahren zu ihrer Herstellung sowie ihre Verwendung zur Herstellung von Arzneimitteln zur Behandlung und/oder Prophylaxe von Krankheiten bei Menschen und Tieren, insbesondere von kardiovaskulären Erkrankungen.

Ein Anstieg der intrazellulären Calcium-Konzentration ist ein Hauptauslöser für die Kontraktion der Gefäßmuskulatur (Somlyo, A.P. und Himpens, B. FASEB J. 1989, 3, 2266-2276). Dies geschieht in erster Linie durch Agonisten wie z.B. Phenylephrin oder Thromboxan A2, die nach Stimulierung der Phosphatidylinositolkaskade die Freisetzung von Calcium aus dem sarkoplasmatischen Retikulum bewirken. Die Erhöhung des intrazellulären Calciums aktiviert die MLC-Kinase (Myosin-Leichte-Ketten-Kinase), die die MLC-Untereinheiten des Myosinmoleküls phosphoryliert (Kamm, K.H. und Stull, J.T., Annu. Rev. Pharmacol. Toxicol. 1985, 25, 593-603). MLC-Phosphorylierung induziert die Glattmuskelkontraktion, MLC-Dephosphorylierung nach einer Reduktion der intrazellulären Calciumkonzentration resultiert in der Relaxation des Gefäßes.

Neben der Calcium-abhängigen MLC-Phosphorylierung existiert noch ein weiterer zentraler aber Calcium-unabhängiger Regulationsmechanismus des Gefäßtonus. Hierbei handelt es sich um den Rho/Rho-Kinase-Signalweg (Noda, M. et al., FEBS Lett. 1995, 367, 246-250; IJehata, M. et al., Nature 1997, 389, 990-994; Fukata, Y. et al., Trends in Pharmacological Sciences 2001, 22, 32-39). Binden Agonisten wie z.B. Phenylephrin oder Thromboxan A2 an ihre Rezeptoren, so führt dies zur Aktivierung der kleinen G-Proteine Rho, die dann mit der Rho-Kinase interagieren und diese aktivieren. Die aktivierte Rho-Kinase inhibiert die Myosin-Phosphatase, nachdem sie eine Untereinheit des Enzyms phosphoryliert hat. Gleichzeitig phosphoryliert Rho-Kinase MLC an der Stelle, die auch von der MLC-Kinase phosphoryliert wird. Eine Hemmung der Myosin-Phosphatase sowie der Phosphorylierung von MLC induziert die Kontraktion der Gefäßmuskulatur. Im Gegensatz dazu führt eine Hemmung der Rho-Kinase zu einer Gefäßrelaxation. Inhibitoren der Rho-Kinase bewirken daher eine Senkung des Blutdruckes und eine Steigerung des koronaren Blutflusses.

Darüber hinaus führten Inhibitoren der Rho-Kinase zu einer Hemmung des Wachstums von Tumorzellen und Metastasen (Itoh et al. Nat. Med. 1999, 5, 221; Somlyo et al. Biochem. Biophys. Res. Commun. 2000, 269, 652) und inhibieren die Angiogenese (Uchida et al. Biochem. Biophys. Res. Commun. 2000, 269, 633; Gingras et al. Biochem. J. 2000, 348 Bd. 2, 273).

Den erfindungsgemäßen Verbindungen ähnliche Strukturen sind lediglich aus anderen Indikationen bekannt. So offenbart beispielsweise US 2001/0020030 A1 substituierte Thienopyridine und Thienopyrimidine zur Behandlung von inflammatorischen Erkrankungen, WO 02/32872 offenbart stickstoffenthaltende aromatische Ringverbindungen als Inhibitoren der Neovaskularisation.

Gegenstand der vorliegenden Erfindung sind Verbindungen der Formel 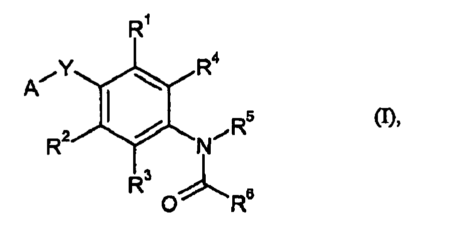 worin
- A: einen Rest 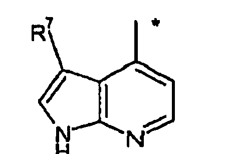 bedeutet,
worin
R⁷ für Wasserstoff, Chlor oder Methyl steht,
und
* für die Anknüpfstelle an Y steht,
- Y: O bedeutet,
- R¹ und: R² unabhängig voneinander Wasserstoff, Fluor oder Chlor bedeuten,
- R³ und: R⁴ unabhängig voneinander Wasserstoff oder Fluor bedeuten,
- R⁵: Wasserstoff bedeutet,
- R⁶: einen Rest bedeutet, der ausgewählt ist aus der Gruppe von:
(C₁-C₆)-A1kyl, das durch Amino, Hydroxy, (C₁-C₆)-Alkoxy, (C₁-C₆)-Alkylthio, (C₁-C₆)-Alkylamino, (C₅-C₆)-Cycloalkylamino, (C₁-C₆)-Aäkycarbonyl-amino,(C₁-C₆)-Alkoxycarbonyl, Phenyl, 5- oder 6-gliedriges Heteroaryl oder 5- oder 6-gliedriges Heterocyclyl substituiert ist,
worin Alkylamino, Cycloalkylamino oder Phenyl ihrerseits durch Hydroxy, Halogen, (C₁-C₃)-Alkoxy, (C₁-C₃)-Alkylaminooder Phenyl substituiert sein können,
(C₁-C₆)-Alkoxy, das durch Amino oder (C₁-C₆)-Alkylamino substituiert sein kann,

Cyclopentyl, Cyclohexyl, 5- oder 6-gliedrigem Heterocyclyl oder 5- oder 6-gliedrigem Heterocyclyloxy,
wobei Cyclopentyl, Cyclohexyl, Heterocyclyl oder Heterocyclyloxy durch Amino, Hydroxy, (C₁-C₃)-Alkyl, Oxo oder Benzyloxy substituiert sein können,
und Phenyl, Thienyl, Furyl, Pyrrolyl, Pyrazolyl, Thiazolyl, Oxazolyl, Imidazolyl, Pyridyl, Pyrimidyl oder Pyridazinyl,
wobei Phenyl, Thienyl, Furyl, Pyrrolyl, Pyrazolyl, Thiazolyl, Oxazolyl, Imidazolyl, Pyridyl, Pyrimidyl oder Pyridazinyl durch Amino, Hydroxy, Halogen, Cyano, (C₁-C₃)-Alkyl, das seinerseits durch Amino oder (C₁-C₆)-Alkylamino substituiert sein kann, (C₁-C₃)-Allcoxy oder (C₁-C₃)-Alkoxycarbonyl substituiert sein können,
und ihre Salze, Hydrate, Hydrate der Salze und Solvate.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausfilhrungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ehansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure, Trifluoressigsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabiethylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Ein Rahmen der vorliegenden Erfindung haben die Substituenten, soweit nicht anders spezifiziert, die folgende Bedeutung:

Alkyd per und "Alk" und "Alkyl" in Alkoxy, Alkylthio, Alkylamino, Alkylcarbonylamino und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-PYopyl, Isopropyl, tert-Butyl, n-Pentyl und n-Iiexyl.

Alkoxy steht beispielhaft und vorzugsweise für Methoxy, Ethoxy, n-Propoxy. Isopropoxy, tert.-Butoxy, n-Pentoxy und n-Hexoxy.

Alkythio steht beispielhaft und vorzugsweise für Methylthio, Ethylthio, n-Propylthio, Isopropylthio, tert.-Butylthio, n-Pentylthio und n-Hexylthio.

Alkylamino steht für einen Alkylaminorest mit einem oder zwei (unabhängig voneinander gewählten) Alkylsubstituenten. (C₁-C₃)-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent. Beispielhaft und vorzugsweise seien genannt:Methylamino, Ethylamino, n-Propylamino, Isopropylamino, tert.-Butylamino, n-Pentylamino, n-Hexylamino, *N*,*N*-Dimethylamino, *N*,*N*-Diethylamino, *N*-Ethyl-*N*-methylamino, *N*-Methyl-*N*-n-propylanino, *N*-Isopropyl-*N*-n-propylamino, *N*-t-Butyl-*N*-methylamino, *N*-Ethyl-*N*-n-pentylamino und *N*-n-Hexyl-*N*-methylamino.

Alklcarbonylamino steht beispielhaft und vorzugsweise für Methylcarbonylamino, Ethylcarbonylamino, n-Propylcarbonylamino, Isopropylcarbonylamino, tert.-Butylcarbonylamino, n-Pentylcarbonylamino und n-Hexylcarbonylamino.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Cycloalkyl steht für eine Cycloallcylgruppe mit in der Regel 3 bis 8, bevorzugt 5 bis 7 Kohlenstoffatomen, beispielhaft und vorzugsweise für Cyclopropyl, Cyclobutyl, Cyclopentyl, Cyclohexyl und Cycloheptyl.

Cycloalkylamino steht für eine Cycloalkylaminogruppe mit in der Regel 3 bis 8, bevorzugt 5 bis 7 Kohlenstoffatomen, beispielhaft und vorzugsweise für Cyclopropylamino, Cyclobutylamino, Cyclopentylamino, Cyclohexylamino und Cycloheptylamino.

Aryl steht für einen mono- bis tricyclischen aromatischen, carbocyclischen Rest mit in der Regel 6 bis 14, vorzugsweise 6 oder 10, Kohlenstoffatomen; beispielhaft und vorzugsweise für Phenyl, Naphthyl und Phenanthrenyl.

Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Pyrazolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.

Heterocyclyl per se und in Heterocyclyloxy steht für einen mono- oder polycyclischen, vorzugsweise mono- oder bicyclischen, nicht-aromatischen heterocyclischen Rest mit 5 bis 10, in der Regel 5 bis 8, vorzugsweise 5 oder 6 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, wie beispielhaft und vorzugsweise Tetrahydrofuran-2-yl, Tetrahydrofuran-3-yl, Tetrahydrothienyl, Pyrrolidin-2-yl, Pyrmlidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Perhydroazepinyl, Piperazin-1-yl, Piperazin-2-yl.

Heterocyclyloxy steht beispielhaft und vorzugsweise für Tetrahydrofuranyloxy, Tetrahydrothienyloxy, Pyrrolidinyloxy, Pyrrolinyloxy, Pyranyloxy, Piperidinyloxy, Thiopyranyloxy, Morpholinyloxy, Perhydroazepinyloxy, Piperazinyloxy.

Halogen steht für Fluor, Chlor, Brom und Jod.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Besonders bevorzugt sind Verbindungen der Formel (I),
worin
- A: einen Rest 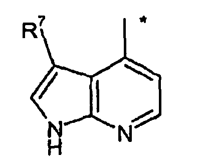 bedeutet,
worin
R⁷ für Wasserstoff, Chlor oder Methyl steht,
und
* für die Anknüpfstelle an Y steht,
- Y: O bedeutet,
- R¹ und R²: unabhängig voneinander Wasserstoff oder Fluor bedeuten,
- R³ und R⁴: Wasserstoff bedeuten,
- R⁵: Wasserstoff bedeutet,
- R⁶: einen Rest bedeutet, der ausgewählt ist aus der Gruppe von:
(C₁-C₆)-Alkyl, das durch Amino, Hydroxy, (C₁-C₆)-Alkylamino, Cyclohexylamino oder Piperidinyl substituiert ist,
worin Alkylamino oder Cyclohexylamino ihrerseits durch Hydroxy oder Phenyl substituiert sein können,
(C₁-C₆)-Alkoxy, das durch Amino oder (C₁-C₆)-Alkylamino substituiert sein kann,
Cyclopentyl, Piperazinyl, Piperidinyl, Pyrrolidinyl, Piperidinyloxy oder Pyrrolidinyloxy,
wobei Cyclopentyl, Piperazinyl, Piperidinyl, Pyrrolidinyl, Piperidinyloxy oder Pyrrolidinyloxy durch Amino, Hydroxy, (C₁-C₃)-Alkyl oder Benzyloxy substituiert sein können,
und Phenyl oder Thienyl,
wobei Phenyl oder Thienyl durch (C₁-C₃)-Alkyl, das seinerseits durch Amino oder (C₁-C₆)-Alkylamino substituiert sein kann, substituiert sein können,
und ihre Salze, Hydrate, Hydrate der Salze und Solvate.

Ebenfalls besonders bevorzugt sind Verbindungen der Formel (I), worin
- A: einen Rest 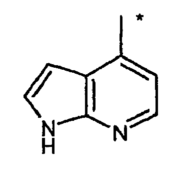 bedeutet, worin * für die Anknüpfstelle an Y steht.

Gegenstand der vorliegenden Erfindung sind auch Verbindungen der Formel (I), worin
- A: einen Rest 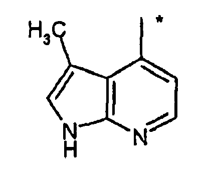 bedeutet, worin * für die Anknüpfstelle an Y steht.

Ebenfalls besonders bevorzugt sind Verbindungen der Formel (I), worin Y Sauerstoff bedeutet.

Ebenfalls besonders bevorzugt sind Verbindungen der Formel (I), worin R¹ Fluor, R² Wasserstoff oder Fluor und R³ und R⁴ Wasserstoff bedeuten.

Ebenfalls besonders bevorzugt sind Verbindungen der Formel (I), worin R⁵ Wasserstoff bedeutet.

Ebenfalls besonders bevorzugt sind Verbindungen der Formel (I), worin der Rest R⁶ durch eine Amino- oder Hydroxygruppe und/oder durch einen Heterocyclus, der mindestens ein Stickstoffatom im Ring enthält, substituiert ist.

Ganz besonders bevorzugt sind Kombinationen von zwei oder mehreren der oben genannten Vorzugsbereiche.

Weiterer Gegenstand der vorliegenden Erfindung ist ein Verfahren zur Herstellung der Verbindungen der Formel (I), das dadurch gekennzeichnet ist, dass man
entweder
**[A]** Verbindungen der Formel 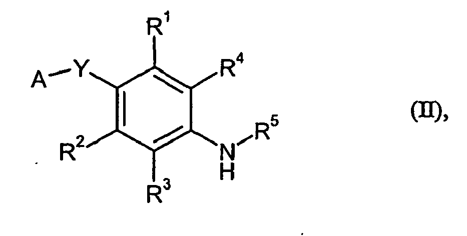 worin
   A, Y, R¹, R², R³, R⁴ und R⁵ die oben angegebene Bedeutung aufweisen,
   mit Verbindungen der Formel 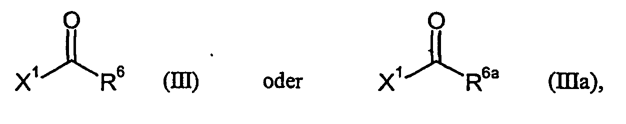 worin
   - R⁶: die oben angegebene Bedeutung aufweist,
   - R^{6a}: einem wie oben definierten Rest R⁶ entspricht, der aber anstelle einer sekundären oder tertiären Aminogruppe einen Chlorsubstituenten oder anstelle einer freien Aminogruppe eine Nitrogruppe oder eine - beispielsweise mit einer tert-Butyloxycarbonylgruppe - geschützte Aminogruppe enthält und
   - X¹: für Halogen, bevorzugt Chlor oder Brom, oder Hydroxy steht,
   umsetzt
   und im Fall der Umsetzung mit Verbindungen (IIIa) anschließend noch im Rest R^{6a} den Chlorsubstituenten durch ein Amin substituiert, die Nitrogruppe zur entsprechenden Aminogruppe hydriert oder die Schutzgruppe - beispielsweise eine tert-Butyloxycarbonylschutzgruppe - zur Freisetzung der entsprechenden freien Aminogruppe abspaltet
   oder
**[B]** Verbindungen der Formel 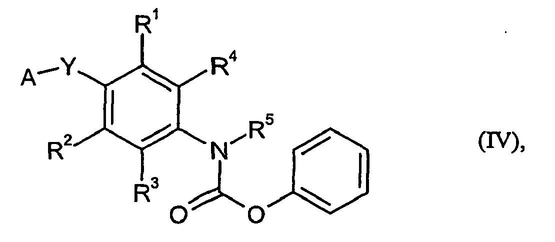
worin
A, Y, R¹, R², R³, R⁴ und R⁵ die oben angegebene Bedeutung aufweisen,
mit Verbindungen der Formel

H₂N-R⁸ (V),

worin
R⁸ die oben angegebene Bedeutung aufweist,
zu Verbindungen der Formel (I) umsetzt.

Im Verfahrensschritt [A] für den Fall, dass X¹ für Halogen steht, erfolgt die Umsetzung im Allgemeinen in einem inerten Lösungsmittel, gegebenenfalls in Gegenwart einer Base, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, 2-Butanon, Dimethylsulfoxid, Acetonitril oder Pyridin, bevorzugt sind Tetrahydrofuran oder Methylenchlorid.

Basen sind beispielsweise Alkalihydroxide wie Natrium- oder Kaliumhydroxid, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropylethylamin, bevorzugt Diisopropylethylamin oder Triethylamin.

Im Verfahrensschritt [A] für den Fall, dass X¹ für Hydroxy steht, erfolgt die Umsetzung im Allgemeinen in einem inerten Lösungsmittel in Gegenwart von üblichen Kondensationsmitteln, gegebenenfalls in Gegenwart einer Base, bevorzugt in einem Temperaturbereich von Raumtemperatur bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, Dimethylsulfoxid, Acetonitril oder Pyridin, bevorzugt sind Tetrahydrofuran, Dimethylformamid oder Methylenchlorid.

Übliche Kondensationsmittel sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-l-ethoxycarbonyl-1,2-dihydrochinolin, oder Propanphosphonsäureanhydrid, oder Isobutylchlorofonnat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluroniumhexafluorophosphat (HBTU), 2-(2-Oxo-1-(2H)-pyridyl)-1,1,3,3-tetramethyluroniumterafluoro-borat (TPTU) oder O-(7-Azabenzotriazol-1-yl)-N,N,N',N'-tetramethyluroniumhexafluorophosphat (HATU), oder 1-Hydroxybenzotriazol (HOBt), oder Benzotriazol-1-yloxytris(dimethylamino)phosphoniunmhexafluoro-phosphat (BOP), oder 1-Chlor-N,N-2-trimethylpropenylamin, oder Mischungen aus diesen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4-Dimethylaminopyridin oder Diisopropylethylamin.

Bevorzugt ist die Kombination von N-(3-Dimethylaminoisopropyl)-N'-ethylcarbodiimid-Hydrochlorid (EDC), 1-Hydroxybenztriazol (HOBt) und Diisopropylethylamin in Methylenchlorid.

Die im Verfahrensschritt [A] gegebenenfalls erfolgende Substitution des Chlorsubstituenten durch ein Amin, Reduktion der Nitrogruppe zur entsprechenden Aminogruppe oder Abspaltung Aminoschutzgruppe zur Freisetzung der entsprechenden freien primären oder sekundären Aminogruppe erfolgt jeweils unter üblichen, dem Fachmann geläufigen Bedingungen. In diesem Zusammenhang sei auf beispielhafte Reaktionsbedingungen bei den entsprechenden Herstellungsbeispielen im experimentellen Teil verwiesen.

Im Verfahrensschritt [B] erfolgt die Umsetzung zu Verbindungen der Formel (I) im Allgemeinen in einem inerten Lösungsmittel, bevorzugt in einem Temperaturbereich von Raumtemperatur bis zur Rückflusstemperatur des Lösungsmittels bei Normaldruck.

Inerte Lösungsmittel sind beispielsweise Halogenkohlenwasserstoffe wie Methylenchlorid oder Ether wie Methyl-tert.-butylether, Dioxan, Tetrahydrofuran, Glykoldimethylether oder Diethylenglykoldimethylether, oder andere Lösungsmittel wie Dimethylformamid, Dimethylacetamid, Dimethylsulfoxid oder Acetonitril, bevorzugt ist Dimethylformamid.

Zur Herstellung der Verbindungen der Formel (II) aus Verfahrensschritt [A] werden beispielsweise entweder
**[A1]** Verbindungen der Formel 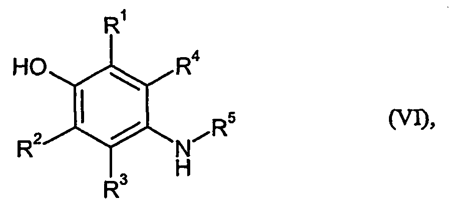 worin
   R¹, R², R³ und R⁴ die oben angegebene Bedeutung aufweisen,
   mit Verbindungen der Formel

   A-X² (VII) oder A-X² (VIIa),

   worin
   - A: die oben angegebene Bedeutung aufweist
   - A': einem wie oben definierten Rest A entspricht, der zusätzlich einen Chlorsubstituenten enthält und
   - X²: für Halogen, bevorzugt Fluor oder Chlor, oder Nitro steht,
   umgesetzt
   und im Fall der Umsetzung mit Verbindungen (VIIa) anschließend noch der Chlorsubstituent im Rest A' durch katalytische Hydrierung in einen Wasserstoffsubstituenten überführt
   oder
**[A2]** Verbindungen der Formel 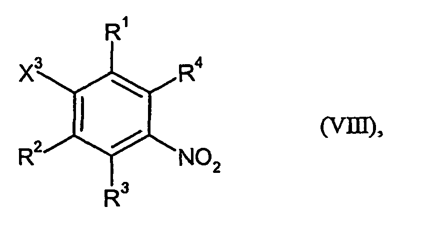 worin
   R¹, R², R³ und R⁴ die oben angegebene Bedeutung aufweisen, und
   - x³: für Halogen, bevorzugt Brom oder Iod, steht,
   in einer ersten Stufe mit Verbindungen der Formel

   A-NH₂ (IX),

   worin
   - A: die oben angegebene Bedeutung aufweist,
   umgesetzt und anschließend in einer zweiten Stufe die Nitrogruppe nach üblichen, dem Fachmann bekannten Methoden zur entsprechenden Aminogruppe reduziert und dann die so erhaltene Aminogruppe gegebenenfalls noch durch Umsetzung mit Verbindungen der Formel

   X⁴—R⁵ (X),

   worin
   - R⁵: für (C₁-C₆)-Alkyl steht und
   - X⁴: für Halogen, bevorzugt Chlor oder Brom, steht,
nach üblichen, dem Fachmann bekannten Methoden alkyliert.

Im Verfahrensschritt [A1] erfolgt die Umsetzung im Allgemeinen in einem inerten Lösungsmittel wie beispielsweise N,N-Dimethylformamid, N-Methylpyrrolidon oder Dimethylsulfoxid in Gegenwart einer Base wie beispielsweise Alkalicarbonate, wie z.B. Natrium- oder Kaliumcarbonat, oder anderen Basen, wie z.B. Kalium-tert.-butylat, Kalium-bis-(trimethylsilyl)amid oder Natriumhydrid, bei einer Temperatur von 60°C bis zur Rückflusstemperatur des Lösungsmittels bei Normaldruck.

Im Verfahrensschritt [A2] erfolgt die Umsetzung im Allgemeinen unter Bedingungen der Buchwald-Reaktion, beispielsweise mit Kalium-tert-butylat, Tris(dibenzylidenaceton)-dipalladium(0) [Pd₂(dba)₃] und Bis(diphenyl-phosphino)ferrocen in Toluol bei einer Temperatur von 100°C bei Normaldruck.

Darüber hinaus können Verbindungen der Formel (II) nach üblichen, dem Fachmann geläufigen Methoden weiter derivatisiert werden, wie beispielsweise im Experimentellen Teil bei den Beispielen 6A bis 9A und 15A angegeben.

Die Verbindungen der Formel (IV) lassen sich beispielsweise durch Umsetzung von Verbindungen der Formel (II) mit Chlorameisensäurephenylester nach Verfahren [A] herstellen.

Die Verbindungen der Formel (III), (IIIa), (V), (VI), (VII), (VIIa), (VIII), (IX) und (X) sind dem Fachmann an sich bekannt oder lassen sich nach üblichen literaturbekannten Verfahren herstellen.

Die Herstellung der erfindungsgemäßen Verbindungen kann durch folgendes Syntheseschema verdeutlicht werden.

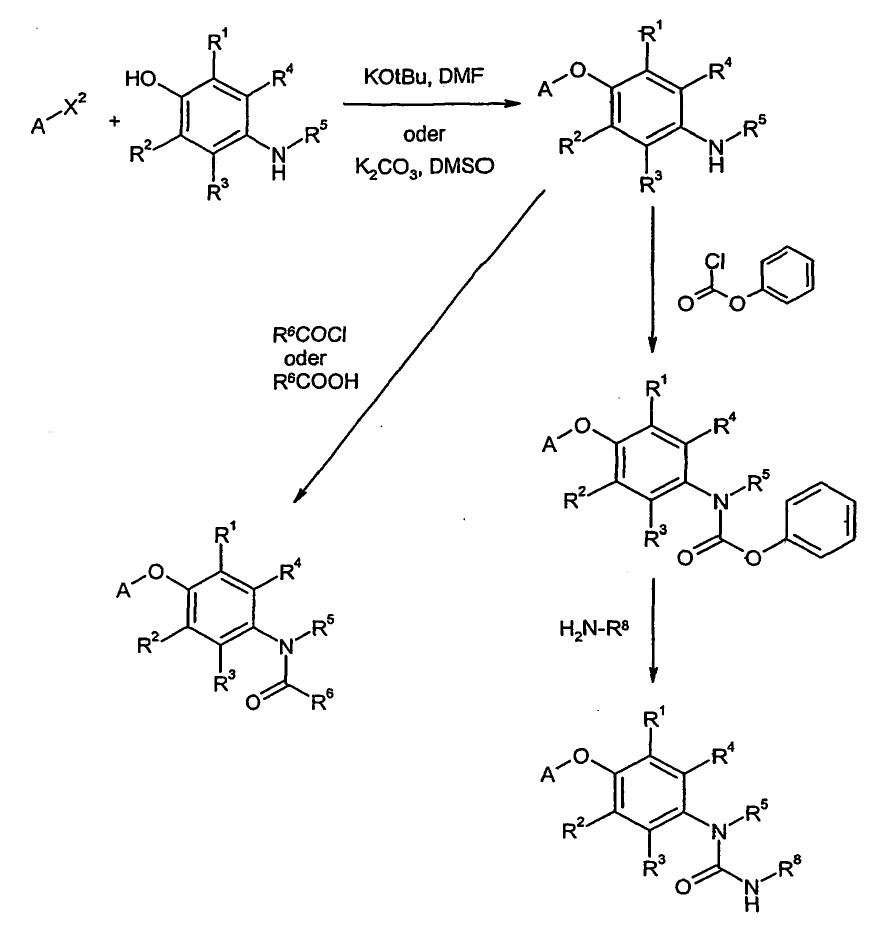

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen lässt sich durch ihre Wirkung als Rho-Kinase-Inhibitoren erklären.

Möglich ist der Einsatz der erfindungsgemäßen Verbindungen zur Behandlung und/oder Prophylaxe von Erkrankungen, vorzugsweise von kardiovaskulären Erkrankungen.

Die erfindungsgemäßen Verbindungen sind geeignet für die Prophylaxe und/oder Behandlung von kardiovaskulären Erkrankungen wie beispielsweise Bluthochdruck und Herzinsuffizienz, stabiler und instabiler Angina pectoris, peripheren und kardialen Gefäßerkrankungen, von Arrhythmien, von thromboembolischen Erkrankungen und Ischämien wie Myokardinfarkt, Hirnschlag, transitorischen und ischämischen Attacken, peripheren Durchblutungsstörungen, Subarachnoidalblutungen, Verhinderung von Restenosen wie beispielsweise nach Thrombolysetherapien, percutanen transluminalen Angioplastien (PTA), percutanen transluminalen Koronarangioplastien (PTCA), Bypass sowie zur Prophylaxe und/oder Behandlung von Arteriosklerose, Alzheimer, Niereninsuffizienz, Glaukom, asthmatischen Erkrankungen, COPD und Krankheiten des Urogerütalsysterns wie beispielsweise Prostatahypertrophie, erektiler Dysfunktion, weiblicher sexueller Dysfunktion, Osteoporose, Gastroparese und Inkontinenz.

Weiterhin können die erfindungsgemäßen Verbindungen zur Prophylaxe und/oder Behandlung von Krebserkrankungen, insbesondere von Tumoren eingesetzt werden.

Im Rahmen der vorliegenden Erfindung umfasst die Definition von Tumoren sowohl benigne, wie auch maligne Tumore und damit beispielsweise auch benigne Neoplasien, Dysplasien, Hyperplasien, wie auch Neoplasien mit Metastasenbildung. Weitere Beispiele für Tumore sind Karzinome, Sarkome, Karzinosarkome, Tumore der blutbildenden Organe, Tumore des Nervengewebes z.B. des Gehirns oder Tumore von Hautzellen. Bei der Tumorbildung kommt es zur unkontrollierten oder unzureichend kontrollierten Zellteilung. Der Tumor kann örtlich begrenzt sein, er kann aber auch das umliegende Gewebe infiltrieren und sich dann durch das lymphatische System oder durch den Blutstrom an einem neuen Ort festsetzen. Somit gibt es primäre und sekundäre Tumore. Primäre Tumore sind ursprünglich in dem Organ entstanden, in dem sie gefunden werden. Sekundäre Tumore haben sich durch Metastasenbildung in einem anderen Organ festgesetzt und sich dann an ihrem neuen Ort ausgebreitet.

Ebenfalls möglich ist die Verwendung der erfindungsgemäßen Verbindungen zur Prophylaxe und/oder Behandlung von Erkrankungen, insbesondere der zuvor genannten Krankheitsbilder.

Weiterer Gegenstand der vorliegenden Erfindung ist die Verwendung der erfindungsgemäßen Verbindungen zur Herstellung eines Arzneimittels zur Prophylaxe und/oder Behandlung von Erkrankungen, insbesondere der zuvor genannten Krankheitsbilder.

Weiterer Gegenstand der vorliegenden Erfindung sind Arzneimittel, enthaltend eine erfmdungsgemäße Verbindung in Kombination mit einem oder mehreren weiteren Wirkstoffen, insbesondere zur Prophylaxe und/oder Behandlung der zuvor genannten Erkrankungen.

Die erfindungsgemäße Verbindung kann systemisch und/oder lokal wirken. Zu diesem Zweck kann sie auf geeignete Weise appliziert werden, wie z.B. oral, parenteral, pulmonal, nasal, sublingual, lingual, buccal, rectal, transdermal, conjunctival, otisch, als Stents oder als Implantat.

Für diese Applikationswege kann die erfindungsgemäße Verbindung in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten, sich verzögert auflösenden oder unlöslichen Überzügen, die die Freisetzung der erfindungsgemäßen Verbindungen kontrollieren), in der Mundhöhle schnell zerfallende Tabletten oder Filme/Oblaten, Kapseln, Dragees, Granulate, Pellets, Pulver, Emulsionen, Suspensionen, Aerosole oder Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalatiorrsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder, Stents oder Implantate.

Die erfindungsgemäßen Verbindungen können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich sowohl in der Human- als auch in der Veterinärmedizin als vorteilhaft erwiesen, die erfindungsgemäße Verbindung in Gesamtmengen von etwa 0.01 bis etwa 700, vorzugsweise 0.01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält die erfindungsgemäße Verbindung vorzugsweise in Mengen von etwa 0.1 bis etwa 80, insbesondere 0.1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- DC: Dünnschichtchromatographie
- DCI: direkte chemische Ionisation (bei MS)
- DCM: Dichlormethan
- DIEA: *N*,*N*-Diisopropylethylamin
- DMA: *N*,*N*-Dimethylacetamid
- DMF: *N*,*N*-Dimethylformamid
- DMSO: Dimethylsulfoxid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- h: Stunde
- HATU: *O*-(7-Azabenzotriazol-1-yl)-*N*,*N*,*N*'*N*'-tetramethyluroniumhexafluorphosphat
- HOAT: 3H-[1,2,3]-Triazol[4,5-b]pyridin-3-ol
- HOBt: 1-Hydroxy-1H-benzotriazol x H₂O
- HPLC: Hochdruck-, Hochleistungsflüssigchromatographie
- konz.: konzentriert
- LC-MS: Flüssigchromatographie-gekoppelte Massenspektroskopie
- LDA: Lithiumdiisopropylamid
- min.: Minuten
- MPLC: Mitteldruck-, Mittelleistungsflüssigchromatographie
- MS: Massenspektroskopie
- NMR: Kernresonanzspektroskopie
- org.: organisch
- proz.: prozentig
- RF: Rückfluss
- R_{f}: Retentionsfaktor (bei DC)
- RP-HPLC: Reverse Phase HPLC
- RT: Raumtemperatur
- Rₜ: Retentionszeit (bei HPLC)
- TFA: Trifluoressigsäure
- THF: Tetrahydrofuran

### HPLC-, LCMS- und GCMS-Methoden:

### Methode 1 (LCIMS)

Gerätetyp MS: Micromass ZQ; Gerätetyp HPLC: Waters Alliance 2795; Säule: Phenomenex Synergi 2µ Hydro-RP Mercury 20 mm x 4mm; Eluent A: 1 1 Wasser + 0.5 ml 50%ige Ameisensäure, Eluent B: 1 1Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

### Methode 2 (LC/MS)

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

### Methode 3 (LC/MS)

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2:5 min/3.0 min/4.5 min. 2 ml/min; Ofen: 50°C; LTV-Detektion: 210 nm.

### Methode 4 (LC/MS)

Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50%ige Ameisensäure; Eluent B: Acetonitril + 500 µl 50%ige Ameisensäure / l; Gradient: 0.0 min 5%B → 2.0 min 40%B → 4.5 min 90%B → 5.5 min 90%B; Ofen: 45°C; Fluss: 0.0 min 0.75 ml/min → 4.5 min 0.75 ml/min 5.5 min → 5.5 min 1.25 ml/min; UV-Detektion: 210 nm.

### Methode 5 (LC/MS)

Instrument: Micromass Platform LCZ mit HPLC Agilent Serie 1100; Säule: Thermo HyPURITY Aquastar 3µ 50 mm x 2.1 mm; Eluent A: 1 1 Wasser + 0.5 ml 50%ige Ameisensäure, Eluent B: 11 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; IN-Detektion: 210 nm.

### Methode 6 (LCIMS)

Gerätetyp MS: Micromass ZQ; Gerätetyp HPLC: Waters Alliance 2795; Säule: Merck Chromolith SpeedROD RP-18e 50 mm x 4.6mm; Eluent A: Wasser + 500 µl 50%ige Ameisensäure / 1; Eluent B: Acetonitril + 500 µl 50%ige Ameisensäure / 1; Gradient: 0.0 min 10%B→ 3.0 min 95%B→ 4.0 min 95%B; Ofen: 35°C; Fluss: 0.0 min 1.0 ml/min → 3.0 min 3.0 ml/min→ 4.0 min 3.0 ml/min; UV-Detektion: 210 nm.

### Methode 7 (HPLC)

Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 6.5 min 90%B; Fluss: 0.75 ml/min; Temp.: 30°C; UV-Detektion: 210 nm.

### Präparative HPLC

Säule: YMC Gel ODS-AQ S-5 / 15 µM, 250 mm x 30 mm, Eluent A: Wasser, Eluent B: Acetonitril; Gradient: 0.00 min 30%B → 3.00 min 30%B → 34.0 min 95%B → 38.0 min 30%B; Temp.: Raumtemperatur; Fluss: 50 ml/min; UV-Detektion.

### Ausgangsverbindungen

### Beispiel 1A

### 1H-Pyrrolo[2,3-b]pyridin-7-oxid

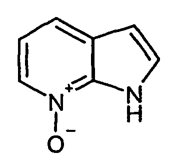

540 g (2.35 mol) 3-Chlorperbenzoesäure werden in 6.11 1 Dichlormethan gelöst und abgeschiedenes Wasser wird abgetrennt. Die organische Phase wird über Natriumsulfat getrocknet und auf 0°C gekühlt. Dann gibt man eine Lösung aus 163 g (1.38 mol) 1H-Pyrrolo[2,3-b]pyridin in 1.00 1 Dichlormethan zu und lässt die Temperatur auf Raumtemperatur ansteigen. Nach 2 Stunden gibt man soviel Methanol zu, dass sich der gebildete Niederschlag wieder auflöst. Die Mischung wird über Kieselgel filtriert (Eluens: Dichlormethan/Methanol 95:5) und die Produktfraktionen nach Einengen am Hochvakuum getrocknet.

Ausbeute: 145 g (75% d. Th.)
HPLC (Methode 7): Rₜ = 2.02 min.
MS (ESI pos.): m/z = 135 (M+H)⁺, 152 (M+NH₄)⁺, 269 (2M+H)⁺

¹H-NMR (DMSO-d₆, 200 MHz): δ= 6.58 (d, 1H), 7.07 (dd, 1H), 7.48 (d, 1H), 7.65 (d, 1H), 8.17 (d, 1H), 12.42-12.64 (br. s, 1H).

### Beispiel 2A

### 4-Nitro-1H-pyrrolo[2,3-b]pyridin-7-oxid

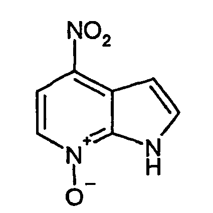

Eine Durchführung größerer Ansätze als die fünffache Menge der beschriebenen Größe ist aufgrund der Ergebnisse der Differentialthermoanalyse nicht empfehlenswert.

Eine Lösung von 20.0 g (149 mmol) 1H-Pyrrolo[2,3-b]pyridin-7-oxid (aus Beispiel 1A) in 160 ml Trifluoressigsäure wird auf Raumtemperatur gekühlt. Anschließend tropft man langsam 69.3 ml 65%ige Salpetersäure zu und lässt 2 h bei Raumtemperatur rühren. Man gießt auf Eis und stellt mit 45%iger Natriumhydroxidlösung einen pH-Wert von 8-9 ein. Der Niederschlag wird abgesaugt und mit Wasser gewaschen. Es werden die Rohprodukte aus 4 Ansätzen der beschriebenen Größe und einem analog durchgeführten 13 g-Ansatz vereinigt und gemeinsam gereinigt. Die Rohprodukte werden in Wasser aufgeschlämmt und mit 2N Natriumhydroxidlösung auf pH 8-9 eingestellt. Nach 10 min Rühren wird der Niederschlag abgesaugt und im Hochvakuum getrocknet.

Ausbeute: 29.7 g (24% d. Th.)
HPLC (Methode 7): Rₜ = 3.02 min.
MS (ESI pos.): m/z =180 (M+H)⁺, 197 (M+NH4)⁺, 359 (2M+H)⁺

¹H-NMR (DMSO-d₆, 200 MHz): δ = 7.03 (d, 1H), 7.80 (d, 1H), 8.03 (d, 1H), 8.31 (d, 1H), 13.22-13.41 (br. s, 1H).

### Beispiel 3A

### 6-Chlor-4-nitro-1H-pyrrolo[2,3-b]pyridin

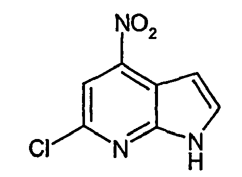

5.00 g (27.9 mmol) 4-Nitro-1H-pyrrolo[2,3-b]pyridin-7-oxid (aus Beispiel 2A) und 11.8 ml (55.8 mmol) Hexamethyldisilazan werden in 290 ml THF unter Argonatmosphäre vorgelegt. 10.8 ml (140 mmol) Chlorameisensäuremethylester werden bei RT zugegeben. Die Lösung wird über Nacht unter RT gerührt. Die Reaktionslösung wird über eine Kieselgelkartusche filtriert und mit Dichlormethan/Methanol 10:1 1 nachgewaschen.

Ausbeute: 2.8 g (70% d. Th.)
LC-MS (Methode 4): Rₜ = 2.74 min.
MS (ESI pos.): m/z = 198 (M+H)⁺

¹H-NMR (DMSO-d₆, 200 MHz): δ = 7.00 (mc, 1H), 7.97 (s, 1H), 8.00 (t, 1H), 12.79 (s, 1H).

### Beispiel 4A

### {4-[(6-Chlor-1H-pyrrolo[2,3-b]pyridin-4-yl)oxy]-3-fluorphenyl}amin

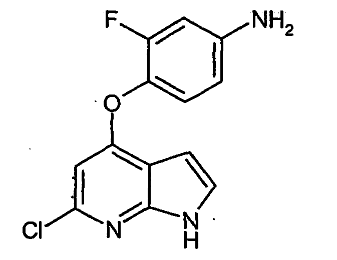

4-Amino-2-fluorphenol (0.77 g, 6.07 mmol) wird in DMF gelöst. Kalium-tert-butylat (0.68 g, 6.07 mmol) wird zugegeben und das Gemisch wird 30 Minuten bei Raumtemperatur gerührt. Dann werden nacheinander gepulvertes Kaliumcarbonat (0.35 g, 2.53 mmol) und 1.00 g (5.06 mmol) 6-Chlor-4-nitro-1H-pyrrolo[2,3-b]pyridin (aus Beispiel 3A) dazugegeben. Das Gemisch wird 12 Stunden bei 120°C gerührt. Nach dem Abkühlen wird es mit Essigsäureethylester (200 ml) verdünnt. Die Suspension wird über Celite^{®} abgesaugt und mit Essigsäureethylester nachgewaschen. Die Lösung wird nacheinander mit wässriger Natriumhydrogencarbonatlösung und Natriumchloridlösung ausgeschüttelt. Die organische Phase wird über wasserfreiem Magnesiumsulfat getrocknet und eingeengt. Der Rückstand wird durch Säulenchromatographie gereinigt (Kieselgel 60, Eluens: Dichlormethan : Aceton = 5 : 1).

Ausbeute: 0.95 g (67% d. Th.)
LC-MS (Methode 6): Rₜ = 1.99 min.
MS (ESIpos): m/z = 278 (M+H)⁺

### Beispiel 5A

### [3-Fluor-4-(1H-pyrrolo[2,3-b]pyridin-4-yloxy)phenyl]amin

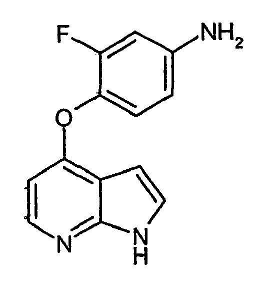

3.2 g (11.5 mmol) {4-[(6-Chlor-1H-pyrrolo[2,3-b]pyridin-4-yl)oxy]-3-fluorphenyl}amin (aus Beispiel 4A) werden in Ethanol bei 50°C gelöst. Dann lässt man die Lösung auf RT abkühlen und gibt 2.45 g (2.30 mmol) 10% Palladium auf Aktivkohle hinzu. Das Gemisch wird über Nacht unter 2 bar Wasserstoff-Druck hydriert. Das Palladium wird anschließend über Kieselgur abgesaugt, mit Ethanol nachgewaschen und das Filtrat eingeengt.

Ausbeute: 2.5 g (89% d.Th.)
LC-MS (Methode 4): Rₜ = 2.43 min.
MS (ESI pos.): m/z = 244 (M+H)⁺

¹H-NMR (DMSO-d₆, 200 MHz): δ = 5.45 (mc, 2H), 6.25 (mc, 2H), 6.40-6.55 (br. 2H), **7.05** (t, 1H), 7.33 (mc, 1H), 8.25 (d, 1H), 11.69 (s, 1H).

### Beispiel 6A

### [3-Fluor-4-({1-[(4-methylphenyl)sulfonyl]-1H-pyrrolo[2,3-b]pyridin-4-yl}oxy)phenyl]amin

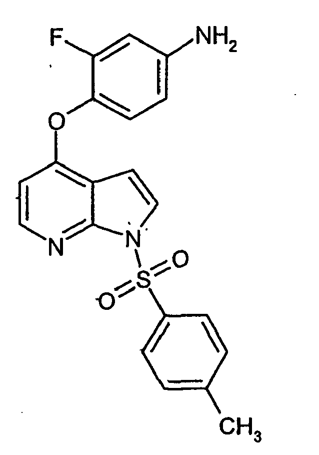

998 mg (4.10 mmol) [3-Fluor-4-(1H-pyrrolo[2,3-b]pyridin-4-yloxy)phenyl]amin (aus Beispiel 5A) werden in 50 ml THF gelöst, mit 230 mg (5.74 mmol) Natriumhydrid (in THF) versetzt und anschließend eine Stunde bei RT gerührt. Dann werden 860 mg (4.51 mmol) p-Toluolsulfonsäurechlorid dazugegeben und die Reaktionslösung für eine Stunde bei 60°C weitergerührt. Die Suspension wird über Celite^{®} filtriert und mit THF und etwas Dichlormethan/Methanol 10:1 nachgewaschen und das Lösungsmittel im Vakuum entfernt. Der Rückstand wird als Rohprodukt weiter umgesetzt.

Ausbeute: 1.65g (86% d. Th.)
LC-MS (Methode 1): Rₜ = 2.39 min.

### Beispiel 7A

### N-[3-Fluor-4-({1-[(4-methylphenyl)sulfonyl]-1H-pyrrolo[2,3-b]pyridin-4-yl}oxy)phenyl]acetamid

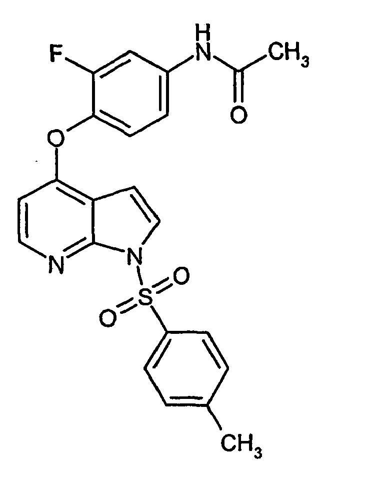

3.02 g (7.60 mmol) [3-Fluor-4-({1-[(4-methylphenyl)sulfonyl]-1H-pyrrolo[2,3-b]pyridin-4-yl}oxy)phenyl]amin (aus Beispiel 6A) werden in 30 ml Acetanhydrid gelöst und eine Stunde bei 50°C gerührt. Dann werden flüchtige Bestandteile im Vakuum entfernt und überschüssiges Reagens mehrmals mit Toluol azeotrop entfernt. Das Rohprodukt wird über eine Kieselgelsäule aufgereinigt (Eluens: Cyclohexan : Essigsäureethylester 1:1).

Ausbeute: 2.04 g (58% d. Th.)
LC-MS (Methode 3): Rₜ = 2.50 min.
MS (ESI pos.): m/z = 440 (M+H)⁺

¹H-NMR (DMSO-d₆, 400 MHz): δ. = 2.07 (s, 3H), 2.35 (s, 3H), 6.55 (m, 1H), 6.66 (m, 1H), 7.34 (mc, 2H), 7.43 (d, 2H), 7.80 (m, 2H), 8.01 (d, 2H), 8.20 (d, 1H), 10.26 (s, 1H).

### Beispiel 8A

### N-[4-({3-Brom-1-[(4-methylphenyl)sulfonyl]-1H-pyrrolo[2,3-b]pyridin-4-yl}oxy)-3-fluorphenyl]acetamid

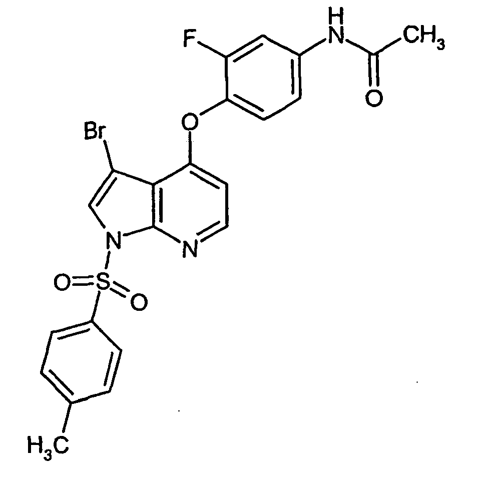

490 mg (1.11 mmol) N-[3-Fluor-4-({1-{(4-methylphenyl)sulfonyl]-1H-pyrrolo[2,3-b]pyrdin-4-yl}oxy)phenyl]acetamid (aus Beispiel 7A) werden in 35 ml Dichlormethan gelöst und auf 0°C gekühlt. Dazu gibt man 114 µl (2.23 mmol) Brom. Nach einer Stunde gibt man Eis hinzu sowie 10%ige Natriumthiosulfatlösung. Nach Extraktion mit Dichlormethan wird die organische Phase über Magnesiumsulfat getrocknet und das Lösungsmittel im Vakuum entfernt. Man reinigt durch Chromatographie an Kieselgel (Eluens: Dichlormethan : Aceton : 10:1).

Ausbeute: 360 mg (62% d. Th.)
LC-MS (Methode 1): Rₜ = 2.50 min.

¹H-NMR (DMSO-d₆, 400 MHz): δ = 2.07 (s, 3H), 2.36 (s, 3H), 6.50 (m, 1H), 7.34 (mc, 2H), 7.44 (d, 2H), 7.80 (m, 1H), 8.02 (d, 2H), 8.08 (s, 1H), 8.23 (d, 1H), 10.23 (s, 1H).

### Beispiel 9A

### N-[3-Fluor-4-({3-methyl-1-[(4-methylphenyl)sulfonyl]-1H-pyrrolo[2,3-b]pyridin-4-yl} oxy)phenyl]acetamid

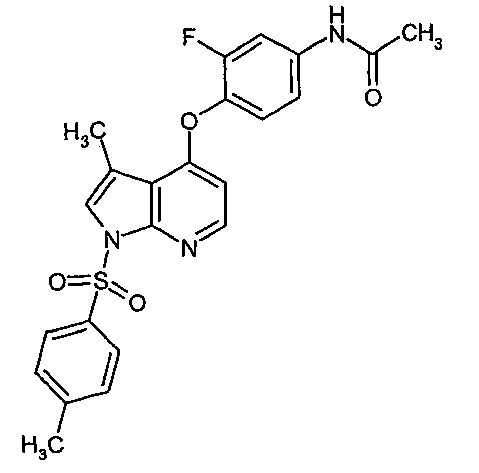

200 mg (0.39 mmol) N-[4-({3-Brom-1-[(4-methylphenyl)sulfonyl]-1H-pyrrolo[2,3-b]pyridin-4-yl}oxy)-3-fluorphenyl]acetamid (aus Beispiel 8A) und 97 mg (1.16 mmol) Natriumhydrogencarbonat werden in einer Mischung aus Dimethoxyethan (10 ml) und Wasser (3 ml) suspendiert, und es wird entgast. Man gibt 15.7 mg (0.02 mmol) [1,1'-Bis-(diphenylphosphino)-ferrocen]-palladium(II)-chlorid-Methylendichlorid-Komplex und 107 µl (0.77 mmol) Trimethylboroxin zu und erhitzt zwei Stunden auf 85°C. Zur Aufarbeitung wird das Reaktionsgemisch mit 2 ml Dichlormethan/Methanol 10:1 über eine Kieselgel-Extrelutkartusche filtriert und das Lösungsmittel im Vakuum entfernt. Der Rückstand wird durch präparative HPLC gereinigt.

Ausbeute: 83 mg (47% d. Th.)
LC-MS (Methode 1): Rₜ = 2.43 min.

¹H-NMR (DMSO-d₆, 200 MHz): δ = 2.08 (s, 3H), 2.35 (s, 3H), 2.39 (m, 3H), 6:40 (d, 1H), 7.34 (m, 2H), 7.41 (d, 2H), 7.62 (d, 1H), 7.78 (m, 1H), 7.95 (d, 2H), 8.14 (d, 1H), 10.22 (s, 1H).

### Beispiel 10A

### 3-Methyl-1H-pyrrolo[2,3-b]pyridin-7-oxid

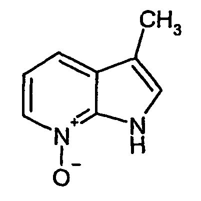

Analog zu Beispiel 1A wird die Titelverbindung durch Oxidation von 11.0 g (54.1 mmol) 3-Methyl-1H-pyrrolo[2,3-b]pyridin (Hands, D.; Bishop, B.; Cameron, M.; Edwards, T.S.; Cottrell, I.F.; Wright, S.H.B.; Synthesis 1996 (7), 877-882) mit 24.2 g (108.2 mmol) 3-Chlorperbenzaesäure gewonnen.

Ausbeute: 5.4 g (67% d. Th.)
LC-MS (Methode 3): Rₜ = 1.19 min.
MS (ESI pos.): m/z = 149 (M+H)⁺

¹H-NMR (DMSO-d₆, 300 MHz): δ = 2.25 (m, 3H), 7.05 (m, 1H), 7.21 (s, 1H), 7.59 (m, 1H), 8.10 (s, 1H), 12.06 (s, 1H).

### Beispiel 11A

### 4-Chlor-3-methyl-1H-pyrrolo[2,3-b]pyridin

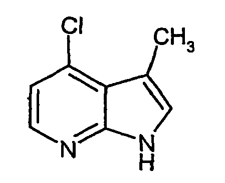

1.00 g (6.75 mmol) 3-Methyl-1H-pyrrolo[2,3-b]pyridin-7-oxid (aus Beispiel 10A) werden in 5 ml Phosphorylchlorid suspendiert. Dann werden 2 ml Chloroform dazugegeben, und das Gemisch wird über Nacht auf Rückflusstemperatur erhitzt. Man lässt auf RT abkühlen und gießt auf Essigsäureethylester/Eiswasser. Anschließend gibt man festes Natriumcarbonat dazu. Die Phasen werden getrennt und die wässrige Phase mit Essigsäureethylester gewaschen. Die organischen Phasen werden über Natriumsulfat getrocknet und eingeengt. Der Rückstand wird durch Säulenchromatographie gereinigt (Kieselgel 60, Eluens: Cyclohexan : Methanol = 4 : 1).

Ausbeute: 200 mg (18% d. Th.)
LC-MS (Methode 3): Rₜ = 2.05 min.

¹H-NMR (DMSO-d₆, 200 MHz): δ = 2.41 (m, 3H), 7.10 (d, 1H), 7.31 (s, 1H), 8.07 (d, 1H), 12.44 (s, 1H).

### Beispiel 12A

### 4-Chlor-3-methyl-1H-pyrrolo[2,3-b]pyridin-7-oxid

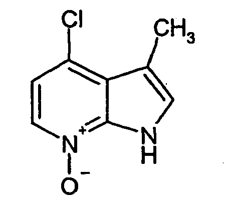

Analog zu Beispiel 1A wird die Titelverbindung durch Oxidation von 898 mg (5.39 mmol) 4-Chlor-3-methyl-1H-pyrrolo[2,3-b]pyridin (aus Beispiel 11A) mit 2.42 g (10.78 mmol) 3-Chlorperbenzoesäure gewonnen.

Ausbeute: 688 mg (70% d. Th.)
LC-MS (Methode 3): Rₜ = 1.75 min.
MS (ESI pos.): m/z = 183 (M+H)⁺

¹H-NMR (DMSO-d₆, 200 MHz): δ = 2.41 (m, 3H), 7.10 (d, 1H), 7.30 (s, 1H), 8.07 (d, 1H), 12.44 (s, 1H).

### Bespiel 13A

### Methyl-4,6-dichlor-3-methyl-1H-pyrrolo[2,3-b]pyridin-1-carboxylat

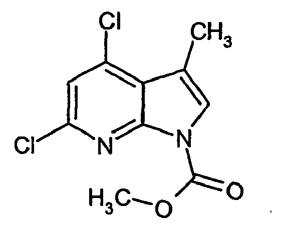

Analog zu Beispiel 3A wird die Titelverbindung aus 688 mg (3.77 mmol) 4-Chlor-3-methyl-1H-pyrrolo[2,3-b]pyridin-7-oxid (aus Beispiel 12A) und 1.78 g (18.84 mmol) Chlorameisensäuremethylester und 0.61 g (3.77 mmol) Hexamethyldisilazan gewonnen.

Ausbeute: 215 mg (22% d. Th.)
LC-MS (Methode 3): Rₜ = 2.44 min.
MS (ESI pos.): m/z = 259 (M+H)⁺

¹H-NMR (DMSO-d₆, 200 MHz): δ = 2.40 (m, 3H), 3.99 (s, 3H), 7.61 (s,1H), 7.77 (d, 1H).

### Beispiel 14A

### {4-[(6-Chlor-3-methyl-1H-pyrrolo[2,3-b]pyridin-4-yl)oxy]-3-fluoiphenyl}amin

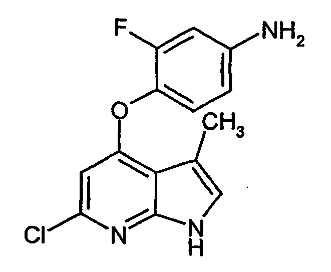

300 mg (1.16 mmol) Methyl-4,6-dichlor-3-methyl-1H-pyrrolo[2,3-b]pyridin-1-carboxylat (aus Beispiel 13A) und 320 mg (2.32 mmol) pulverisiertes Kaliumcarbonat werden in 9 ml DMSO suspendiert. Die Mischung wird entgast und 442 mg (3.48 mmol) 4-Amino-2-fluorphenol werden zugesetzt. Man erhitzt 4 Stunden auf 120°C. Nach Zugabe von Essigsäureethylester wird über Celite^{®} abgesaugt und mit Essigsäureethylester nachgewaschen. Das Filtrat wird dreimal mit gesättigter Natriumhydrogencarbonatlösung und mit gesättigter Natriumchloridlösung geschüttelt. Man trocknet über Natriumsulfat und entfernt das Lösungsmittel im Vakuum. Der Rückstand wird durch Säulenchromatographie gereinigt (Kieselgel 60, Eluens: Dichlormethan : Methanol = 50 : 1).

Ausbeute: 142 mg (42% d. Th.)
LC-MS (Methode 3): Rₜ = 2.32 min.
MS (ESI pos.): m/z = 292 (M+H)⁺

### Bespiel 15A

### {3-Fluor-4-[(3-methyl-1H-pyrrolo[2,3-b]pyridin-4-yl)oxy]phenyl}amin

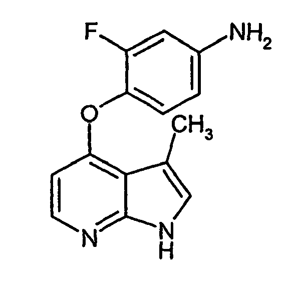

Analog zu Beispiel 5A wird die Titelverbindung durch katalytische Hydrierung von 142 mg (0.49 mmol) {4-[(6-Chlor-3-methyl-1H-pyrrolo[2,3-b]pyridin-4-yl)oxy]-3-fluorphenyl}amin (aus Beispiel 14A) gewonnen.

Ausbeute: 125 mg (100% d.Th.)

### Alternative Herstellungsmethode:

267 mg (0.59 mmol) N-[3-Fluor-4-({3-methyl-1-[(4-methylphenyl)sulfonyl]-1H-pyrrolo[2,3-b]-pyridin-4-yl}oxy)phenyl]acetamid (aus Beispiel 9A) werden in 10 ml Ethanol gelöst. Man gibt 5 ml 20%ige Natronlauge hinzu und erhitzt das Reaktionsgemisch über Nacht auf 90°C. Das Lösungsmittel wird weitgehend im Vakuum entfernt. Der Rückstand wird in Essigsäureethylester aufgenommen und mit Natriumcarbonatlösung geschüttelt. Die organische Phase wird mit Natriumchloridlösung gewaschen, über Magnesiumsulfat getrocknet und das Lösungsmittel im Vakuum entfernt.

Ausbeute: 170 mg (97% d. Th.)
LC-MS (Methode 3): Rₜ = 1.52 min.

¹H-NMR (DMSO-d₆, 300 MHz): δ = 2.41 (s, 3H), 5.38 (s, 2H), 6.08 (d, 1H), 6.40-6.56 (m, 2H), 7.00 (t, 1H), 7.08 (s, 1H), 7.93 (d, 1H), 11.26 (s, 1H).

### Beispiel 16A

### 2-Chlor-N-[3-fluor-4-(1H-pyrrolo[2,3-b]pyridin-4-yloxy)phenyl]acetamid

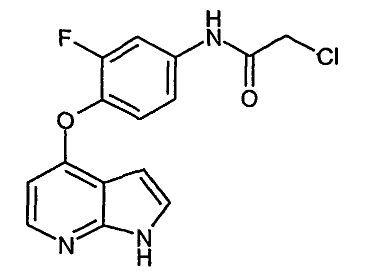

Zu einer Lösung von 1.35 g (4.82 mmol) [3-Fluor-4-(1H-pyrrolo[2,3-b]pyridin-4-yloxy)phenyl]-amin (aus Beispiel 5A) und 1.48 ml (10.6 mmol) Triethylamin in 20 ml Dichlormethan tropft man bei 0°C 0.42 ml (5.30 mmol) Chloracetylchlorid langsam zu. Man lässt 2 Stunden bei 0°C rühren. Man wäscht dann die Reaktionslösung mit gesättigter Natriumcarbonatlösung, trennt die organische Phase ab und entfernt das Lösungsmittel im Vakuum. Man reinigt mittels einer Kieselgelfiltration (Eluens: Essigsäureethylester) und erhält einen Feststoff, der ohne weitere Aufreinigung umgesetzt wird.

Ausbeute: 1.41 g (91% d. Th.)
LC-MS (Methode 1): Rₜ = 1.56 min.
MS (ESI pos.): m/z = 320, 322 (M+H)⁺

### Beispiel 17A

### 2-Chlor-N-{3-fluor-4-[(3-methyl-1H-pyrrolo[2,3-b]pyridin-4-yl)oxy]phenyl}acetamid

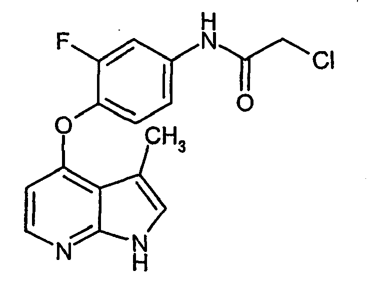

Analog Beispiel 16A wird die Titelverbindung aus 40 mg (0.16 mmol) {3-Fluor-4-[(3-methyl-1H-pyrrolo[2,3-b]pyridin-4-yl)oxy]phenyl}amin (aus Beispiel 15A) und 13.6 µl (0.17 mmol) 2-Chloracetylchlorid synthetisiert.

Ausbeute: 42 mg (52% d. Th.)
LC-MS (Methode 2): Rₜ = 1.87 min.

### Beispiel 18A

### 2-Brom-N-[3-fluor-4-(1H-pyrrolo[2,3-b]pyridin-4-yloxy)-phenyl]propanamid

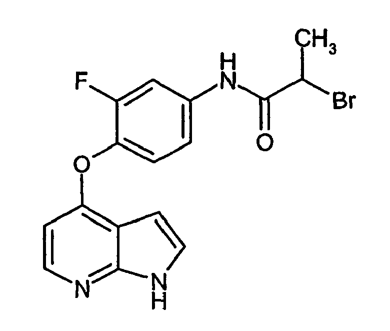

Analog Beispiel 16A wird die Titelverbindung aus 100 mg (0.41 mmol) [3-Fluor-4-(1H-pyrrolo-[2,3-b]pyridin-4-yloxy)phenyl]amin (aus Beispiel 5A) und 95 µl (0.90 mmol) 2-Brompropanoylbromid synthetisiert.

Ausbeute: 173 mg (77% d. Th.)
LC-MS (Methode 1): Rₜ = 1.78 min.
MS (ESI pos.): m/z = 378, 380 (M+H)⁺

### Beispiel 19A

### 4-(Chlormethyl)-N-[3-fluor-4-(1H-pyrrolo[2,3-b]pyridin-4-yloxy)phenyl]benzamid

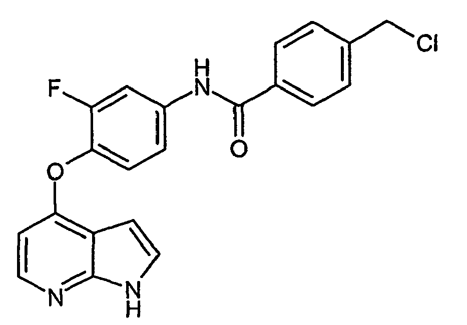

Analog Beispiel 16A wird die Titelverbindung aus 100 mg (0.41 mmol) [3-Fluor-4-(1H-pyrrolo[2,3-b]pyridin-4-yloxy)phenyl]amin (aus Beispiel 5A) und 135 mg (0.71 mmol) 4-Chlormethylbenzoylchlorid synthetisiert.

Ausbeute: 142 mg (65% d. Th.)
LC-MS (Methode 1): Rₜ = 2.11 min.
MS (ESI pos.): m/z = 396, 398 (M+H)⁺

### Beispiel 20A

### Phenyl-[3-fluor-4-(1H-pyrrolo[2,3-b]pyridin-4-yloxy)phenyl]carbamat

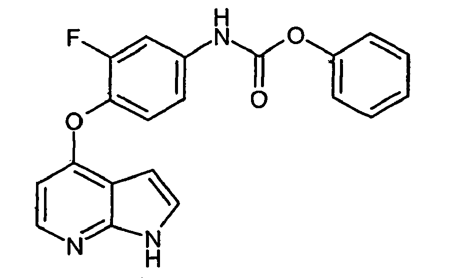

Zu einer Lösung von 80 mg (0.33 mmol) [3-Fluor-4-(1H-pyrrolo[2,3-b]pyridin-4-yloxy)phenyl]-amin (aus Beispiel 5A) in 3.2 ml Essigsäureethylester werden 1.6 ml einer gesättigten Lösung von Natriumhydrogencarbonat in Wasser zugegeben. Zu dieser Suspension werden bei kräftigem Rühren 41 µl (0.33 mmol) Chlorameisensäurephenylester zugetropft und 2 Stunden bei Raumtemperatur gerührt. Man verdünnt mit 10 ml Essigsäureethylester, trennt die Phasen und wäscht die organische Phase mit 5 ml Wasser und gesättigter Natriumchloridlösung. Man entfernt das Lösungsmittel im Vakuum und reinigt mittels einer Kiesegelfiltration (Eluens: Essigsäureethylester). Man erhält einen Feststoff.

Ausbeute: 102 mg (75% d. Th.)
LC-MS (Methode 2): Rₜ = 2.08 min.
MS (ESI pos.): m/z = 364 (M+H)⁺

### Beispiel 21A

### tert-Butyl-[3-({[3-fluor-4-(1H-pyrrolo[2,3-b]pyridin-4-yloxy)phenyl]amino}carbonyl)cyclopentyl]carbamat

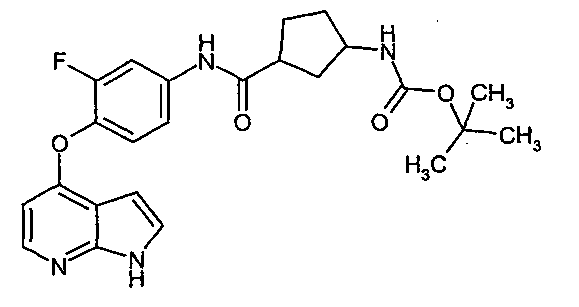

Zu einer Lösung aus 122 mg (0.53 mmol) 3-[(tert-Butoxycarbonyl)amino]cyclopentancarbonsäure und 104 µl (0.94 mmol) N-Methylmorpholin in 5 ml THF tropft man bei -15°C 69 µl (0.53 mmol) Isobutylchlorformiat zu. Man lässt 15 Minuten bei -15°C rühren. Eine Lösung aus 100 mg (0.41 mmol) [3-Fluor-4-(1H-pyrrolo[2,3-b]pyridin-4-yloxy)phenyl]amin (aus Beispiel 5A) in 5 ml THF wird zu der Reaktion bei -15°C hinzugefügt. Es wird 2 Stunden bei 0°C gerührt. Man bricht die Reaktion durch Zugabe von 5 ml Wasser ab, und extrahiert die Suspension mit Essigsäureethylester (dreimal je 10 ml). Man wäscht die organische Phase mit gesättigter Natriumhydrogencarbonatlösung, trocknet über Magnesiumsulfat und entfernt das Lösungsmittel im Vakuum. Der Rückstand wird in 3 ml Methanol gelöst, 22 mg (0.41 mmol) Natriummethanolat werden zugegeben, und die Lösung wird 30 Minuten bei Raumtemperatur gerührt. Man reinigt die Lösung durch präparative HPLC.

Ausbeute: 149 mg (79% d. Th.)
LC-MS (Methode 1): Rₜ = 2.07 min.
MS (ESI pos.): m/z = 455 (M+H)⁺

¹H-NMR (DMSO-d₆, 300 MHz): δ = 1.38 (s, 9H), 1.52 (m, 1H), 1.62 (m, 1H), 1.84 (m, 3H), 2.12 (m, 1H), 2.82 (m, 1H), 3.82 (m, 1H), 6.23 (d, 1H), 6.35 (d, 1H), 6.87 (brd, 1H), 7.33 (t, 1H), 7.37 (m, 2H), 7.83 (dd, 1H), 8.06 (d, 1H), 10.19 (s, 1H), 11.75 (s, 1H).

Analog zu Beispiel 21A werden hergestellt:

| **Bsp.-Nr.** | **Struktur** | **MS, HPLC, LC-MS, ¹H-NMR** |
|---|---|---|
| **22A** | 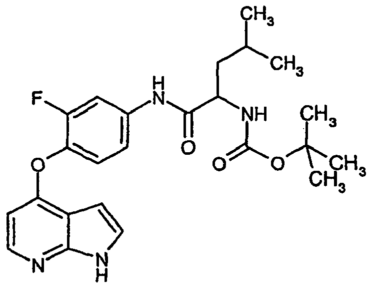 | LC-MS (Methode 1): Rₜ = 2.24 min. MS (ESI pos.): m/z = 457 (M+H)⁺ |
| **23A** | 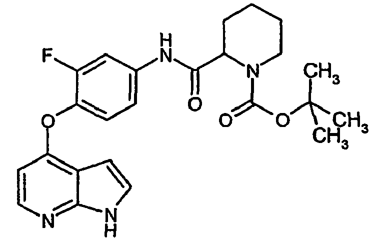 | LC-MS (Methode 3): Rₜ = 2.26 min. MS (ESI pos.): m/z = 455 (M+H)⁺ |
| **24A** | 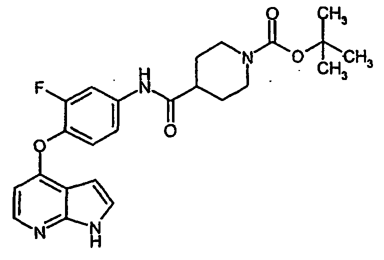 | LC-MS (Methode 1): Rₜ = 2.06 min. MS (ESI pos.): m/z = 455 (M+H)⁺ |
| **25A** | 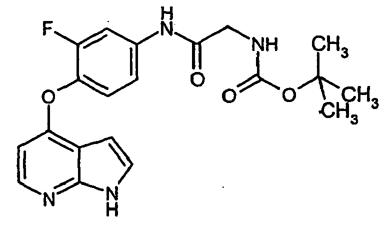 | HPLC (Methode 7): Rₜ = 4.02 min. MS (ESI pos.): m/z = 401 (M+H)⁺ |
| **26A** | 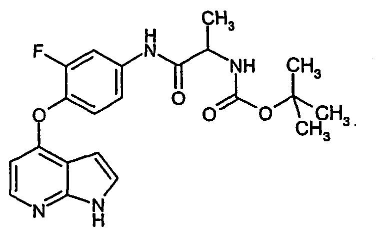 | LC-MS (Methode 1): Rₜ = 1.81 min. MS (ESI pos.): m/z = 415 (M+H)⁺ |
| **27A** | 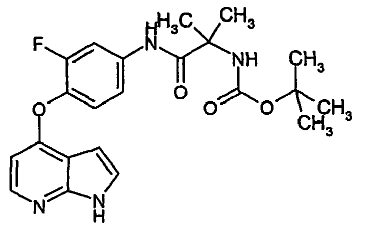 | LC-MS (Methode 1): Rₜ= 1.88 min. MS (ESI pos.): m/z = 429 (M+H)⁺ |
| **28A** | 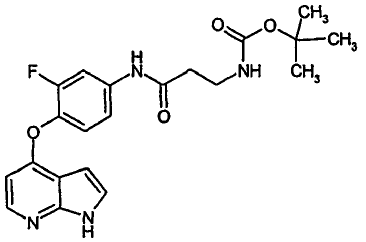 | LC-MS (Methode 3): Rₜ = 1.83 min. MS (ESI pos.): m/z = 415 (M+H)⁺ |
| **29A** | 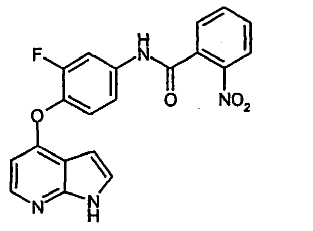 | LC-MS (Methode 1): Rₜ =1.79 min. MS (ESI pos.): m/z = 393 (M+H)⁺ |
| **30A** | 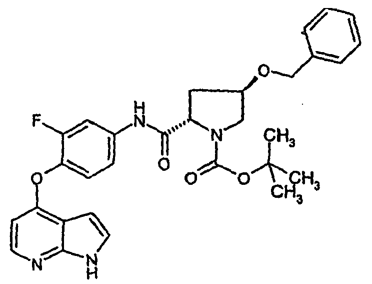 | LC-MS (Methode 1): Rₜ = 2.31 min. MS (ESI pos.): m/z = 547 (M+H)⁺ |
| **31A** | 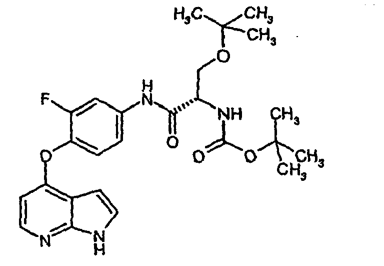 | LC-MS (Methode 1): Rₜ = 2.26 min. MS (ESI pos.): m/z = 487 (M+H)⁺ |

### Beispiel 32A

### tert-Butyl-3-[({[3-fluor-4-(1H-pyrrolo[2,3-b]pyridin-4-yloxy)phenyl]amino}carbonyl)oxy]-pyrrolidin-1-carboxylat

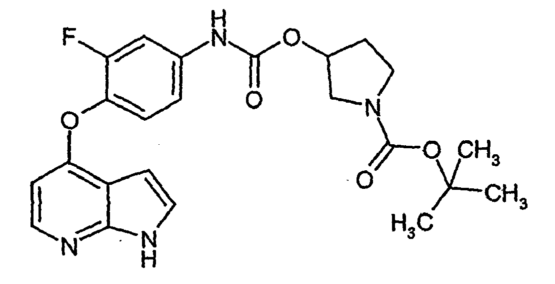

Analog Beispiel 41 wird die Titelverbindung aus 130 mg (0.159 mmol) Phenyl-[3-fluor-4-(1H-pyrrolo[2,3-b]pyridin-4-yloxy)phenyl]carbamat (aus Beispiel 20A) und 35.7 mg (0.190 mmol) tert-Butyl-3-hydroxypyrrolidin-1-carboxylat synthetisiert.

Ausbeute: 36 mg (46% d. Th.)
LC-MS (Methode 2): Rₜ = 2.16 min.
MS (ESI pos.): m/z = 456 (M+H)⁺

### Beispiel 33A

### 4-[(6-Chlor-1H-pyrrolo[2,3-b]pyridin-4-yl)oxy]-3,5-difluoranilin

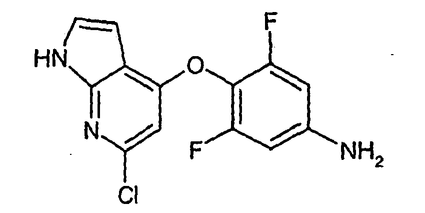

664 mg (3.36 mmol) 6-Chlor-4-nitro-1H-pyrrolo[2,3-b]pyridin (aus Beispiel 3A), 1.39 g (10.1 mmol) pulverisiertes Kaliumcarbonat und 877 mg (5.04 mmol) Natriumdithionit werden in 10 ml DMSO suspendiert. Die Mischung wird entgast, und 915 mg (5.04 mmol) 4-Amino-2,6-difluorphenol-Hydrochlorid werden zugesetzt. Man erhitzt 4 Stunden auf 120°C. Nach Zugabe von Essigsäureethylester wird über Celite abgesaugt und mit Essigsäureethylester nachgewaschen. Das Filtrat wird dreimal mit gesättigter Natriumhydrogencarbonatlösung und mit gesättigter Natriumchloridlösung geschüttelt. Man trocknet über Natriumsulfat und entfernt das Lösungsmittel im Vakuum. Der Rückstand wird durch Säulenchromatographie gereinigt (Kieselgel 60, Eluens: Dichlormethan/Methanol = 50 : 1).

Ausbeute: 356 mg (36% d. Th.)
LC-MS (Methode 1): Rₜ = 2.05 min.
MS (ESI pos.): m/z = 296 (M+H)⁺.

¹H-NMR (DMSO-d₆, 400 MHz): δ = 5.84 (s, 2H), 6.28 (mc, 1H), 6.38-6.41 (m, 3H), 7.42 (mc, 1H), 12.02 (s, 1H).

### Beispiel 34A

### 3,5-Difluor-4-(1H-pyrrolo[2,3-b]pyridin-4-yloxy)anilin

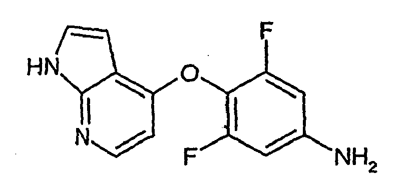

Analog der Synthese von 3-Fluor-4-(1H-pyrrolo[2,3-b]pyridin-4-yloxy)anilin (Beispiel 5A) wird die Titelverbindung durch katalytische Hydrierung aus 408 mg (1.38 mmol) 4-[(6-Chlor-1H-pyrrolo[2,3-b]pyridin-4-yl)oxy]-3,5-difluoranilin (aus Beispiel 33A) gewonnen.

Ausbeute: 360 mg (100% d. Th.)
LC-MS (Methode 1): Rₜ = 1.46 min.
MS (ESI pos.): m/z = 262 (M+H)⁺.

¹H-NMR (DMSO-d₆, 400 MHz): δ = 5.77 (br. s, 1H), 6.28 (dd, 1H), 6.34-6.40 (m, 3H), 7.37 (dd, 1H), 8.06 (d, 1H), 11.76 (br. s, 1H).

### Beispiel 35A

### 2,2,2-Trifluor-N-[3,5-difluor-4-(1H-pyrrolo[2,3-b]pyridin-4-yloxy)phenyl]acetamid

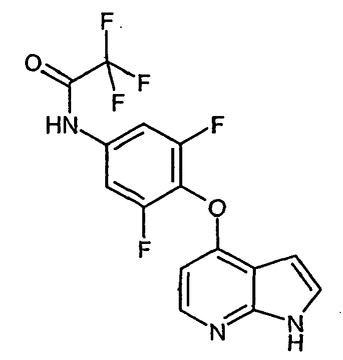

Zu einer Lösung von 200 mg (0.76 mmol) 3,5-Difluor-4-(1H-pyrrolo[2,3-b]pyridin-4-yloxy)anilin und 0.21 ml (1.53 mmol) Triethylamin in wasserfreiem Dichlormethan (20 ml) werden bei 0°C 0.16 ml (1.14 mmol) Trifluoressigsäureanhydrid hinzugetropft. Man lässt 20 min bei 0°C rühren und beendet die Reaktion durch Zutropfen einer gesättigten Natriumhydrogencarbonatlösung (10 ml). Man lässt die Suspension auf RT erwärmen und trennt die Phasen. Die wässrige Phase extrahiert man mit tert.-Butylmethylether (10 ml). Die kombinierten organischen Phasen wäscht man mit einer gesättigten Natriumchloridlösung. Die organische Lösung wird über Magnesiumsulfat getrocknet und eingeengt. Man erhält einen Feststoff, der nicht weiter gereinigt wird.

Ausbeute: 270 mg (98% d. Th.)
HPLC (Methode 3): Rₜ = 2.21 min.
MS (ESI pos.): m/z= 358 (M+H)⁺.

### Beispiel 36A

### N-{4-[(3-Chlor-1H-pyrrolo[2,3-b]pyridin-4-yl)oxy]-3,5-difluorphenyl}-2,2,2-trifluoracetamid

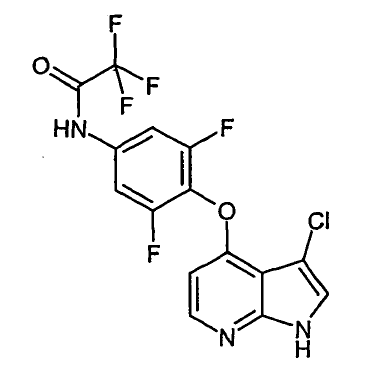

Zu einer Lösung von 250 mg (0.70 mmol) 2,2,2-Trifluor-N-[3,5-difluor-4-(1H-pyrrolo[2,3-b]pyridin-4-yloxy)phenyl]acetamid (aus Beispiel 35A) in wasserfreiem Tetrahydrofuran (5 ml) werden 204 mg (1.54 mmol) N-Chlorsuccinimid und 50 µl 1M wässrige Salzsäure zugegeben. Man lässt die Lösung bei RT über Nacht rühren. Die Titelverbindung fällt aus dem Reaktionsgemisch aus. Durch Absaugen und Trocknen erhält man einen Feststoff.

Ausbeute: 90 mg (33% d. Th.)
HPLC (Methode 3): Rₜ = 2.45 min.
MS (ESI pos.): m/z = 392, 394 (M+H)⁺.

### Beispiel 37A

### 4-[(3-Chlor-1H-pyrrolo[2,3-b]pyridin-4-yl)oxy]-3,5-difluoranilin

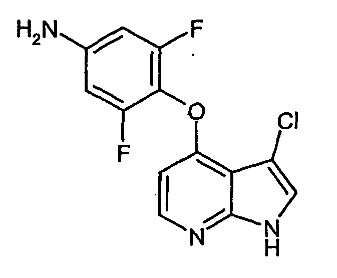

Zu einer Lösung von 90 mg (0.23 mmol) N-{4-[(3-Chlor-1H-pyrrolo[2,3-b]pyridin-4-yl)oxy]-3,5-difluorphenyl}-2,2,2-trifluoracetamid (aus Beispiel 36A) in Ethanol (5 ml) werden 3 ml einer 1N Natriumhydroxid-lösung zugegeben. Man lässt die Reaktion über Nacht rühren. Die Lösung extrahiert man mit tert.-Butylmethylether (zweimal 10 ml). Die kombinierten organischen Phasen wäscht man mit einer gesättigten Natriumchloridlösung. Die organische Phase wird über Magnesiumsulfat getrocknet und eingeengt. Man erhalt einen Feststoff, der nicht weiter gereinigt wird.

Ausbeute: 65 mg (96% d. Th.)
HPLC (Methode 2): Rₜ = 2.13 min.
MS (ESI pos.): m/z = 296, 298 (M+H)⁺.

### Beispiel 38A

### N-[3-Fluor-4-(1H-pyrrolo[2,3-b]pyridin-4-yloxy)phenyl]acetamid

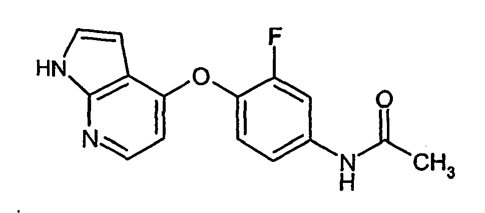

5.00 g (17.9 mmol) 3-Fluor-4-(1H-pyrrolo[2,3-b]pyridin-4-yloxy)anilin (aus Beispiel 5A) werden in 100 ml Dichlormethan suspendiert und auf 0°C gekühlt. Man tropft 9.97 ml (71.5 mmol) Triethylamin und 3.81 ml (53.6 mmol) Acetylchlorid hinzu und lässt noch 2 Stunden bei 0°C rühren. Man gibt gesättigte Natriumhydrogencarbonatlösung hinzu und lässt 10 min bei RT rühren. Dann wird zweimal mit Dichlormethan extrahiert. Unlösliche Bestandteile werden mit etwas Aceton angelöst, man verdünnt mit Dichlormethan und wäscht mit gesättigter Natriumchloridlösung. Die vereinigten organischen Phasen werden über Magnesiumsulfat getrocknet und das Lösungsmittel im Vakuum entfernt. Das Rohprodukt wird in 100 ml Methanol aufgenommen. Man gibt 3.31 ml (17.9 mmol) 5.4 M Natriummethanolatlösung hinzu und lässt 30 min bei RT rühren. Man engt ein und reinigt durch Chromatographie an Kieselgel (Eluens: Dichlormethan/Methanol 100:1 bis 100:5).

Ausbeute: 3.92 g (77% d. Th.)
LC-MS (Methode 3): Rₜ = 1.56 min.
MS (ESI pos.): m/z = 286 (M+H)⁺.

¹H-NMR (DMSO-d₆, 300 MHz): δ = 2.08 (s, 3H), 6.22 (dd, 1H), 6.35 (d, 1H), 7.28-7.38 (m, 3H), 7.80 (dd, 1H), 8.07 (d, 1H), 10.21 (br. s, 1H), 11.72 (br. s, 1H).

### Ausführungsbeispiele

### Beispiel 1

N-[3-Fluor-4-(1H-pyrrolo[2,3-b]pyridin-4-yloxy)phenyl]isonicotinamid

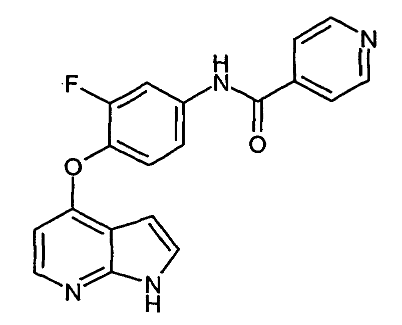

100 mg (0.41 mmol) [3-Fluor-4-(1H-pyrrolo[2,3-b]pyridin-4-yloxy)phenyl]amin (aus Beispiel 5A) und 230 µl (1.64 mmol) Triethylamin werden in 5 ml Dichlormethan gelöst. Man kühlt auf 0°C, setzt 175 mg (1.23 mmol) Isonicotinsäurechlorid hinzu und lässt 24 h bei Raumtemperatur rühren. Dann gibt man Wasser zu und verdünnt mit Dichlormethan, extrahiert und trocknet die organische Phase über Natriumsulfat und entfernt das Lösungsmittel im Vakuum. Der Rückstand wird in 2.5 ml Methanol suspendiert und nach Zugabe von 0.09 ml (0.50 mmol) 5.4-molarer Natriummethanolatlösung 1 h bei Raumtemperatur gerührt. Man reinigt durch präparative HPLC und erhält einen Feststoff.

Ausbeute: 70 mg (49% d. Th.)
LC-MS (Methode 2): Rₜ = 1.52 min.
MS (ESI pos.): m/z = 349 (M+H)⁺

¹H-NMR (DMSO-d₆, 300 MHz): δ = 6.24 (dd, 1H), 6.40 (d, 1H), 7.37 (dd, 1H), 7.42 (t, 1H), 7.64 (dd, 1H), 7.87 (d, 2H), 7.97 (dd, 1H), 8.09 (d, 1H), 8.81 (d, 2H), 10.75 (s, 1H), 11.76 (s, 1H).

### Beispiel 2

### N-[3-Fluor-4-(1H-pyrrolo[2,3-b]pyridin-4-yloxy)phenyl]-1H-pyrrol-2-carboxamid

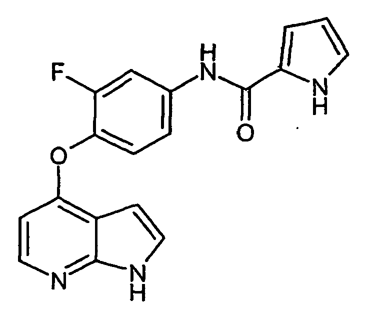

100 mg (0.41 mmol) [3-Fluor-4-(1H-pyrrolo[2,3-b]pyridin-4-yloxy)phenyl]amin (aus Beispiel 5A), 72 µl (0.411 mmol) Diisopropylethylamin und 137 mg (1.23 mmol) Pyrrol-2-carbonsäure werden in 5 ml Dichlormethan gelöst. Man kühlt auf 0°C, setzt 219 mg (1.23 mmol) *N*'-(3-Dimethylaminopropyl)-*N*-ethylcarbodiimid-Hydrochlorid (EDC) hinzu und lässt 24 h bei Raumtemperatur rühren. Dann gibt man Wasser zu und verdünnt mit Dichlormethan, extrahiert und trocknet die organische Phase über Natriumsulfat und entfernt das Lösungsmittel im Vakuum. Der Rückstand wird durch präparative HPLC gereinigt. Man erhält einen Feststoff.

Ausbeute: 46 mg (33% d. Th.)
LC-MS (Methode 2): Rₜ =1.69 min.
MS (ESI pos.): m/z = 337 (M+H)⁺

### Beispiel 3

### N-[3-Fluor-4-(1H-pyrrolo[2,3-b]pyridin-4-yloxy)phenyl]-2-(methylthio)acetamid

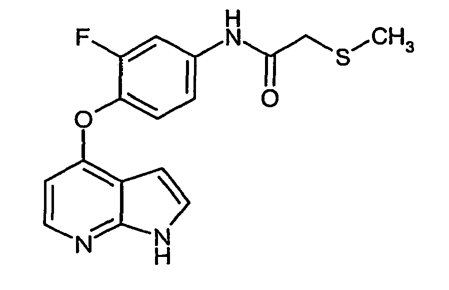

100 mg (0.36 mmol) des Hydrochlorids von [3-Fluor-4-(1H-pyrrolo[2,3-b]pyridin-4-yloxy)-phenyl]amin (aus Beispiel 5A) werden in einer Mischung aus 5.0 ml Dichlormethan und 0.50 ml Pyridin gelöst. Man setzt 89 mg (0.72 mmol) Methylthioessigsäurechlorid hinzu und lässt 20 h bei RT rühren. Man setzt Wasser zu und verdünnt mit Dichlormethan, extrahiert und trocknet die organische Phase über Natriumsulfat und entfernt das Lösungsmittel im Vakuum. Der Rückstand wird in 2.5 ml Methanol suspendiert und nach Zugabe von 0.09 ml (0.50 mmol) 5.4-molarer Natriummethanolatlösung 1 h bei RT gerührt. Man reinigt dann durch präparative HPLC.

Ausbeute: 72 mg (61% d. Th.)
LC-MS (Methode 3): Rₜ =1.63 min.
MS (ESI pos.): m/z = 332 (M+H)⁺

¹H-NMR (DMSO-d₆, 300 MHz): δ = 2.18 (s, 3H), 3.30 (s, 2H), 6.24 (d, 1H), 6.37 (d, 1H), 7.30-7.41 (m, 3H), 7.81 (d, 1H), 8.07 (d, 1H), 10.36 (br. s, 1H), 11.73 (br. s, 1H).

### Beispiel 4

### N²-Acetyl-N¹-[3-fluor-4-(1H-pyrrolo[2,3-b]pyridin-4-yloxy)phenyl]glycinamid

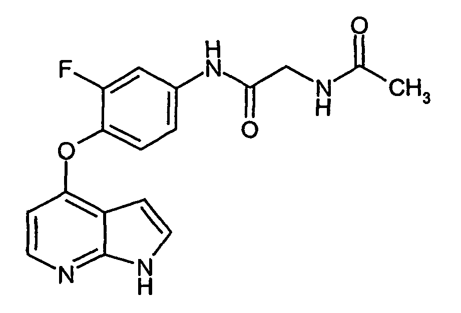

100 mg (0.41 mmol) [3-Fluor-4-(1H-pyrrolo[2,3-b]pyridin-4-yloxy)phenyl]amin (aus Beispiel 5A) werden in einer Mischung aus 5.0 ml Dichlormethan und 0.50 ml Pyridin gelöst. Man setzt 111 mg (0.82 mmol) Acetamidoessigsäurechlorid hinzu und lässt 20 h bei RT rühren. Man setzt Wasser zu und verdünnt mit Dichlormethan, extrahiert und trocknet die organische Phase über Natriumsulfat und entfernt das Lösungsmittel im Vakuum. Der Rückstand wird in 2.5 ml Methanol suspendiert und nach Zugabe von 0.09 ml (0.50 mmol) 5.4-molarer Natriummethanolatlösung 1 h bei RT gerührt. Man reinigt dann durch präparative HPLC und erhält einen Feststoff.

Ausbeute: 66 mg (47% d. Th.)
LC-MS (Methode 1): Rₜ = 1.17 min.
MS (ESI pos.): m/z = 343 (M+H)⁺

¹H-NMR (DMSO-d₆, 200 MHz): δ = 1.90 (s, 3H), 3.89 (d, 2H), 6.21 (d, 1H), 6.36 (d, 1H), 7.31-7.42 (m, 3H), 8.06 (d, 1H), 8.26 (t, 1H), 10.29 (br. s, 1H), 11.76 (br. s, 1H).

### Beispiel 5

### 3-Cyano-N-[3-fluor-4-(1H-pyrrolo[2,3-b]pyridin-4-yloxy)phenyl]benzamid

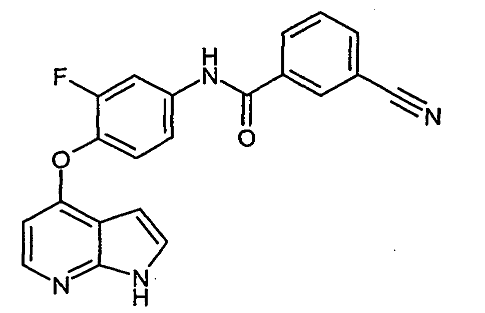

Analog Beispiel 4 werden 93 mg (0.38 mmol) [3-Fluor-4-(1H-pyrrolo[2,3-b]pyridin-4-yloxy)-phenyl]amin (aus Beispiel 5A) mit 127 mg (0.76 mmol) 3-Cyanobenzoylchlorid umgesetzt.

Ausbeute: 93 mg (64% d. Th.)
LC-MS (Methode 3): Rₜ = 1.93 min.
MS (ESI pos.): m/z = 373 (M+H)⁺

¹H-NMR (DMSO-d₆, 200 MHz): δ = 6.26 (dd, 1H), 6.40 (d, 1H), 7.38-7.48 (m, 2H), 7.60-7.68 (m, 1H), 7.79 (t, 1H), 7.99 (dd, 1H), 8.07-8.13 (m, 2H), 8.27 (d, 1H), 8.42 (s, 1H), 10.70 (s, 1H), 11.79 (s, 1H).

### Beispiel 6

### 5-Chlor-N-[3-fluor-4-(1H-pyrrolo[2,3-b]pyridin-4-yloxy)phenyl]-2-methoxybenzamid

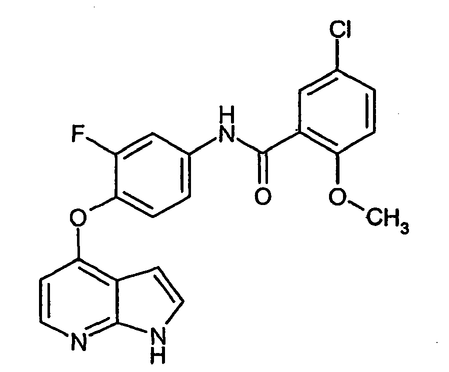

Analog Beispiel 4 werden 100 mg (0.36 mmol) des Hydrochlorids von [3-Fluor-4-(1H-pyrrolo[2,3-b]pyridin-4-yloxy)phenyl]amin (aus Beispiel 5A) mit 147 mg (0.72 mmol) 5-Chlor-2-methoxybenzoesäurechlorid umgesetzt.

Ausbeute: 74 mg (50% d. Th.)
LC-MS (Methode 1): Rₜ = 2.41 min.
MS (ESI pos.): m/z = 412 (M+H)⁺

¹H-NMR (DMSO-d₆, 300 MHz): δ = 3.90 (s, 3H), 6.26 (s, 1H), 6.39 (d, 1H), 7.23 (d, 1H), 7.35-7.42 (m, 2H), 7.52-7.59 (m, 2H), 7.62 (s, 1H), 7.92 (d, 1H), 8.08 (d, 1H), 10.46 (s, 1H), 11.75 (s, 1H).

### Beispiel 7

### 4-({[3-Fluor-4-(1H-pyrrolo[2,3-b]pyridin-4-yloxy)phenyl]amino}carbonyl)benzoesäuremethylester

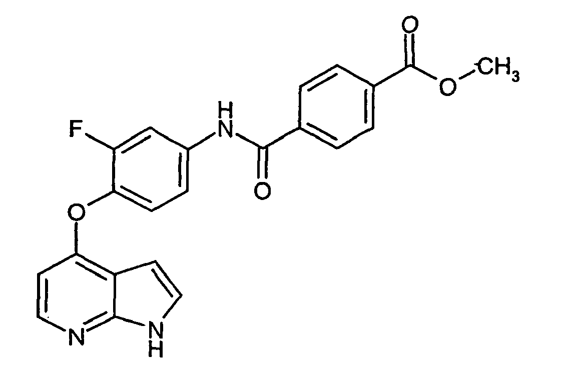

Analog Beispiel 4 werden 100 mg (0.41 mmol) [3-Fluor-4-(1H-pyrrolo[2,3-b]pyridin-4-yloxy)-phenyl]amin (aus Beispiel 5A) mit 163 mg (0.82 mmol) Terephthalsäuremonomethylesterchlorid umgesetzt.

Ausbeute: 7 mg (4% d. Th.)
LC-MS (Methode 2): Rₜ = 2.22 min.
MS (ESI pos.): m/z = 406 (M+H)⁺

¹H-NMR (DMSO-d₆, 400 MHz): δ = 3.91 (s, 3H), 6.25 (d, 1H), 6.40 (d, 1H), 7.38 (d, 1H), 7.42 (t, 1H), 7.64-7.67 (m, 1H), 7.99 (dd, 1H), 8.08-8.14 (m, 5H), 10.72 (br. s, 1H), 11.78 (br. s, 1H).

### Beispiel 8

### N-[3-Fluor-4-(1H-pyrrolo[2,3-b]pyridin-4-yloxy)phenyl]-2-phenylacetamid

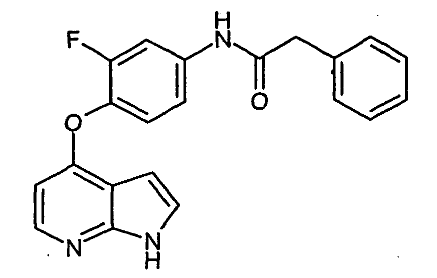

Analog Beispiel 4 werden 100 mg (0.41 mmol) [3-Fluor-4-(1H-pyrrolo[2,3-b]pyridin-4-yloxy)-phenyl]amin (aus Beispiel 5A) mit 127 mg (0.82 mmol) Phenylessigsäurechlorid umgesetzt.

Ausbeute: 89 mg (59% d. Th.)
LC-MS (Methode 1): Rₜ = 1.90 min.
MS (ESI pos.): m/z = 362 (M+H)⁺

¹H-NMR (DMSO-d₆, 200 MHz): δ = 3.67 (s, 2H), 6.22 (dd, 1H), 6.35 (d, 1H), 7.25-7.40 (m, 8H), 7.84 (dd, 1H), 8.06 (d, 1H), 10.48 (s, 1H), 11.76 (s, 1H).

### Beispiel 9

### 2-(4-Chlorphenyl)-N-[3-fluor-4-(1H-pyrrolo[2,3-b]pyridin-4-yloxy)phenyl]acetamid

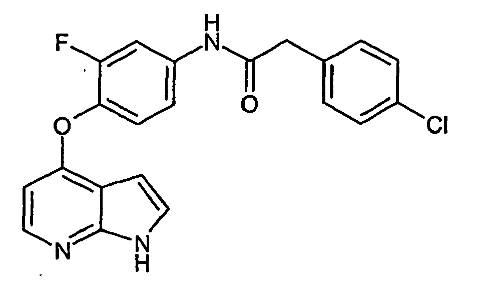

Analog Beispiel 4 werden 100 mg (0.41 mmol) [3-Fluor-4-(1H-pyrrolo[2,3-b]pyridin-4-yloxy)-phenyl]amin (aus Beispiel 5A) mit 155 mg (0.82 mmol) 4-Chlorphenylessigsäurechlorid umgesetzt.

Ausbeute: 60 mg (35% d. Th.)
LC-MS (Methode 1): Rₜ = 2.10 min.
MS (ESI pos.): m/z = 396 (M+H)⁺

¹H-NMR (DMSO-d₆, 200 MHz): δ = 3.69 (s, 2H), 6.22 (d, 1H), 6.35 (d, 1H), 7.29-7.44 (m, 7H), 7.77-7.85 (m, 1H), 8.06 (d, 1H), 10.50 (br. s, 1H), 11.76.

### Beispiel 10

### N-[3-Fluor4-(1H-pyrrolo[2,3-b]pyridin-4-yloxy)phenyl]-2-(3-thienyl)acetamid

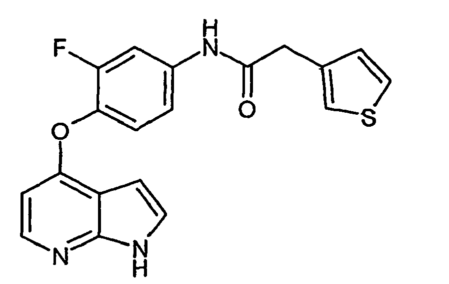

Analog Beispiel 4 werden 100 mg (0.36 mmol) des Hydrochlorids von [3-Fluor-4-(1H-pyrrolo[2,3-b]pyridin-4-yloxy)phenyl]amin (aus Beispiel 5A) mit 115 mg (0.72 mmol) Thiophen-3-essigsäurechlorid umgesetzt.

Ausbeute: 76 mg (58% d. Th.)
LC-MS (Methode 3): Rₜ = 1.90 min.
MS (ESI pos.): m/z = 368 (M+H)⁺

¹H-NMR (DMSO-d₆, 300 MHz): δ = 3.69 (s, 2H), 6.22 (d, 1H), 6.71 (d, 1H), 7.11 (dd, 1H), 7.30-7.41 (m, 4H), 7.50 (dd, 1H), 7.82 (dd, 1H), 8.06 (d, 1H), 10.42 (br. s, 1H), 11.73 (br. s, 1H).

### Beispiel 11

### N-{3-Fluor-4-[(3-methyl-1H-pyrrolo[2,3-b]pyridin-4-yl)oxy]phenyl}-3-methylthiophen-2-carboxamid

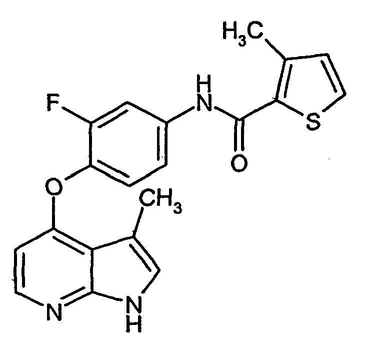

Analog Beispiel 16A wird die Titelverbindung aus 50 mg (0.18 mmol) {3-Fluor-4-[(3-methyl-1H-pyrrolo[2,3-b]pyridin-4-yl)oxy]phenyl}amin (aus Beispiel 15A) und 37.3 mg (0.23 mmol) 3-Methylthiophen-2-carbonsäurechlorid synthetisiert.

Ausbeute: 21 mg (32% d. Th.)
LC-MS (Methode 1): Rₜ = 2.02 min.
MS (ESI pos.): m/z = 368 (M+H)⁺

### Beispiel 12

### N-[3-Fluor-4-(1H-pyrrolo[2,3-b]pyridin-4-yloxy)phenyl]-5-methylthiophen-2-carboxamid

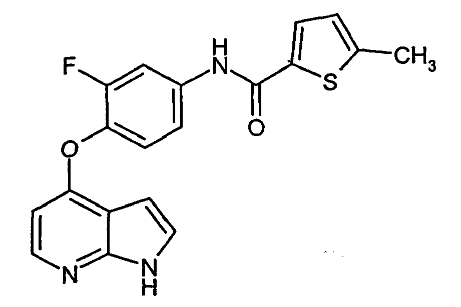

Analog Beispiel 4 werden 100 mg (0.41 mmol) [3-Fluor-4-(1H-pyrrolo[2,3-b]pyridin-4-yloxy)-phenyl]amin (aus Beispiel 5A) mit 132 mg (0.82 mmol) 5-Methylthiophen-2-carbonsäurechlorid umgesetzt.

Ausbeute: 115 mg (76% d. Th.)
LC-MS (Methode 1): Rₜ = 2.05 min.
MS (ESI pos.): m/z = 368 (M+H)⁺

¹H-NMR (DMSO-d₆, 200 MHz): δ = 3.40 (m, 3H), 6.25 (d, 1H), 6.40 (d, 1H), 6.95 (dd, 1H), 7.34-7.43 (m, 2H), 7.54-7.62 (m, 1H), 7.85 (d, 1H), 7.91 (dd, 1H), 8.08 (d, 1H), 10.38 (br. s, 1H), 11.77 (br. s, 1H).

### Beispiel 13

### 3-{[3-Fluor-4-(1H-pyrrolo[2,3-b]pyridin-4-yloxy)phenyl]amino}-3-oxopropansäuremethylester

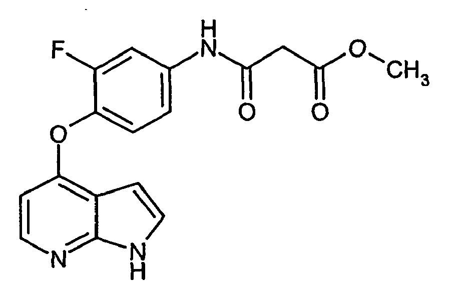

Analog Beispiel 4 werden 100 mg (0.36 mmol) des Hydrochlorids von [3-Fluor-4-(1H-pyrrolo[2,3-b]pyridin-4-yloxy)phenyl]amin (aus Beispiel 5A) mit 98 mg (0.72 mmol) Methylmalonylchlorid umgesetzt.

Ausbeute: 10 mg (8% d. Th.)
LC-MS (Methode 3): Rₜ = 1.51 min.
MS (ESI pos.): m/z = 344 (M+H)⁺

¹H-NMR (DMSO-d₆, 400 MHz): δ = 6.29 (dd, 1H), 6.46 (d, 1H), 7.36-7.43 (m, 3H), 7.77-7.83 (m, 1H), 8.13 (d, 1H), 10.59 (s, 1H), 11.91 (s, 1H).

### Beispiel 14

### N-[3-Fluor-4-(1H-pyrrolo[2,3-b]pyridin-4-yloxy)phenyl]-5-oxo-D-prolinamid

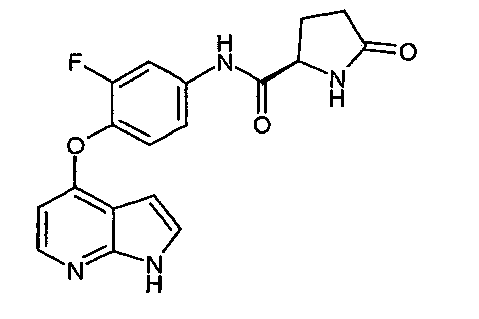

In 2.0 ml THF werden 102 mg (0.79 mmol) D-Pyroglutaminsäure bei 0°C vorgelegt. Man gibt 105 mg (0.79 mmol) 1-Chlor-N,N-2-trimethylpropenylamin hinzu und lässt 2 h bei dieser Temperatur rühren. Dann wird eine Lösung von 100 mg (0.36 mmol) des Hydrochlorids von [3-Fluor-4-(1H-pyrrolo[2,3-b]pyridin-4-yloxy)phenyl]amin (aus Beispiel 5A) in einer Mischung aus 3 ml THF, 1 ml DMF und 0.16 ml 4-Methylmorpholin zugetropft. Man lässt 15 h bei RT rühren, verdünnt dann mit Essigsäureethylester und extrahiert mit Wasser. Die organische Phase wird eingeengt und der Rückstand in Methanol gelöst, mit Natriummethylatlösung versetzt und 1 h bei RT gerührt. Man reinigt mittels präparativer HPLC.

Ausbeute: 10 mg (8% d. Th.)
LC-MS (Methode 3): Rₜ = 1.31 min.
MS (ESI pos.): m/z = 355 (M+H)⁺

¹H-NMR (DMSO-d₆, 300 MHz): δ = 1.97-2.44 (m, 4H), 4.20 (dd, 1H), 6.22 (d, 1H), 6.37 (d, 1H), 7.32-7.46 (m, 3H), 7.83 (dd, 1H), 8.07 (d, 1H), NH (3H) nicht sichtbar.

### Beispiel 15

### 4-[(tert-Butylamino)methyl]-N-[3-fluor-4-(1H-pyrrolo[2,3-b]pyridin-4-yloxy)phenyl]benzamid

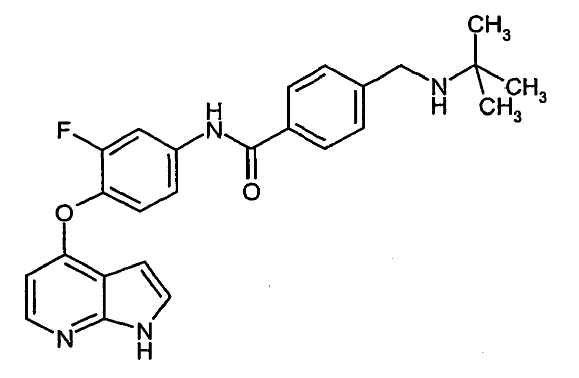

Zu einer Lösung aus 141 mg (0.23 mmol) 2-Chlor-N-[3-fluor-4-(1H-pyrrolo[2,3-b]pyridin-4-yloxy)phenyl]benzamid (aus Beispiel 19A) in 2 ml Dimethylformamid gibt man bei Raumtemperatur 73 µl (0.79 mmol) tert-Butylamin. Man lässt 12 Stunden bei 40°C rühren. Man reinigt die Lösung durch präparative HPLC und erhält einen Feststoff.

Ausbeute: 15 mg (14% d. Th.)
LC-MS (Methode 2): Rₜ = 1.33 min.
MS (ESI pos.): m/z = 433 (M+H)⁺

¹H-NMR (DMSO-d₆, 300 MHz): δ = 1.40 (s, 9H), 4.19 (m, 2H), 6.37 (m, 1H), 6.57 (d, 1H), 7.47 (t, 1H), 7.49 (m, 1H), 7.76 (dd, 1H), 7.77 (d, 2H), 8.05 (dd, 1H), 8.08 (d, 2H), 8.20 (d, 1H), 9.13 (brs, 2H), 10.68 (s, 1H), 12.22 (s, 1H).

### Beispiel 16

### N²-(tert-Butyl)-N¹-[3-fluor-4-(1H-pyrrolo[2,3-b]pyridin-4-yloxy)phenyl]glycinamid

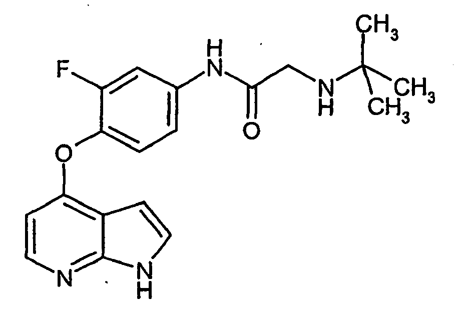

Zu einer Lösung aus 147 mg (0.46 mmol) 2-Chlor-N-[3-fluor-4-(1H-pyrrolo[2,3-b]pyridin-4-yloxy)phenyl]acetamid (aus Beispiel 16A) in 3 ml Dimethylformamid gibt man bei Raumtemperatur 140 µl (1.38 mmol) tert-Butylamin. Man lässt 12 Stunden rühren und reinigt die Lösung durch präparative HPLC und erhält einen Feststoff.

Ausbeute: 85 mg (52% d. Th.)
LC-MS (Methode 1): Rₜ = 1.03 min.
MS (ESI pos.): m/z = 357 (M+H)⁺

¹H-NMR (DMSO-d₆, 300 MHz): δ = 1.07 (s, 9H), 6.22 (m, 1H), 6.36 (d, 1H), 7.35 (m, 2H), 7:47 (dd, 1H), 7.86 (dd, 1H), 8.06 (d, 1H), 11.73 (s, 1H).

Analog zu Beispiel 16 werden hergestellt:

| **Bsp.-Nr.** | **Edukt (Bsp.- Nr.)** | **Struktur** | **MS, HPLC, LC-MS, ¹H-NMR** |
|---|---|---|---|
| **17** | 16A | 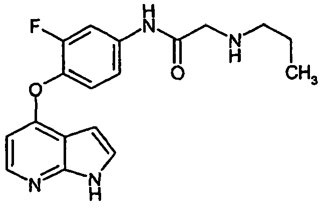 | LC-MS (Methode 2): Rₜ = 1.20 min. MS (ESI pos.): m/z = 345 (M+H)⁺ ¹H-NMR (DMSO-d₆, 300 MHz): δ = 0.89 (t, 3H), 1.45 (q, 2H), 6.23 (m, 1H), 6.35 (d, 1H), 7.34 (t, 1H), 7.37 (m, 1H), 7.46 (dd, 1H), 8.06 (d, 1H), 7.86 (dd, 1H), 11.75 (s, 1H). |
| **18** | 16A | 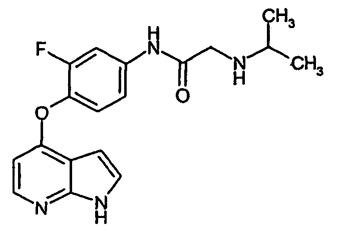 | LC-MS (Methode 1): Rₜ = 0.95 min. MS (ESI pos.): m/z = 343 (M+H)⁺ ¹H-NMR (DMSO-d₆, 300 MHz): δ = 1.01 (d, 6H), 2.74 (m, 1H), 6.23 (m, 1H), 6.35 (d, 1H), 7.34 (t, 1H), 7.37 (m, 1H), 7.46 (dd, 1H), 7.86 (dd, 1H), 8.06 (d, 1H), 11.76 (s, 1H). |
| **19** | 16A | 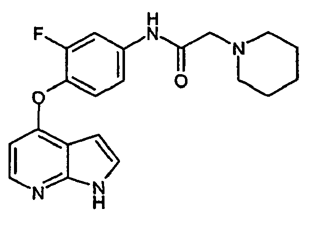 | LC-MS (Methode 1): Rₜ = 0.95 min. MS (ESI pos.): m/z = 343 (M+H)⁺ ¹H-NMR (DMSO-d₆, 300 MHz): δ = 1.41 (m, 2H), 1.57 (m, 4H), 2.46 (m, 4H), 3.09 (s, 2H), 6.23 (m, 1H), 6.35 (d, 1H), 7.34 (t, 1H), 7.37 (m, 1H), 7.50 (dd, 1H), 7.87 (dd, 1H), 8.06 (d, 1H), 9.96 (s, 1H), 11.76 (s, 1H). |
| **20** | 16A | 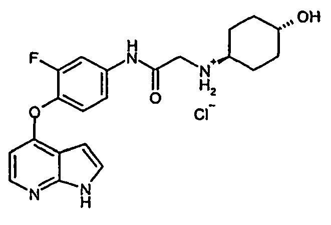 | LC-MS (Methode 1): Rₜ = 0.94 min. MS (ESI pos.): m/z = 399 (M+H)⁺ ¹H-NMR (DMSO-d₆, 300 MHz): δ = 1.20 (m, 2H), 1.43 (m, 2H), 1.89 (m, 2H), 2.02 (m, 2H), 3.06 (m, 1H), 3.36 (m, 1H), 3.99 (s, 2H), 6.30 (m, 1H), 6.50 (d, 1H), 7.46 (m, 3H), 7.83 (dd, 1H), 8.16 (d, 1H), 9.06 (m, 2H), 11.16 (s, 1H), 12.12 (s, 1H). |
| **21** | 16A | 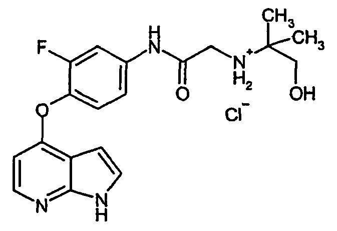 | LC-MS (Methode 1): Rₜ = 0.96 min. MS (ESI pos.): m/z = 373 (M+H)⁺ ¹H-NMR (DMSO-d₆, 300 MHz): δ = 1.26 (s, 6H), 3.06 (m, 1H), 3.16 (s, 1H), 3.50 (s, 2H), 6.35 (m, 1H), 6.55 (d, 1H), 7.48 (m, 3H), 7.84 (dd, 1H), 8.20 (d, 1H), 8.82 (m, 2H), 11.22 (s, 1H), 12.28 (s, 1H). |
| **22** | 16A | 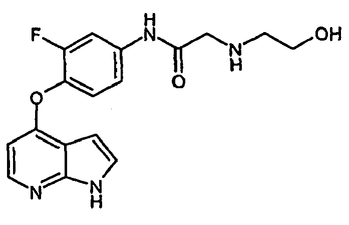 | LC-MS (Methode 1): Rₜ = 0.80 min. MS (ESI pos.): m/z = 345 (M+H)⁺ ¹H-NMR (DMSO-d₆, 200 MHz): δ = 3.71 (t, 3H), 6.34 (m, 1H), 6.54 (d, 1H), 7.47 (m, 3H), 7.84 (dd, 1H), 8.19 (d, 1H), 9.03 (m, 2H), 11.13 (s, 1H), 12.24 (s, 1H). |
| **23** | 17A | 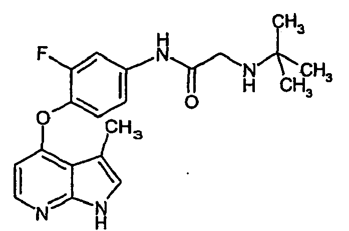 | LC-MS (Methode 1): Rₜ = 1.12 min. |
| **24** | 18A | 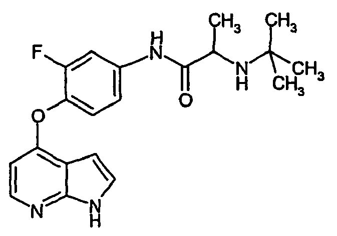 | LC-MS (Methode 1): Rₜ =1.14 min. MS (ESI pos.): m/z = 371 (M+H)⁺ ¹H-NMR (DMSO-d₆, 300 MHz): δ = 1.05 (s, 9H), 1.23 (d, 1H), 3.35 (m, 1H), 6.25 (m, 1H), 6.35 (d, 1H), 7.33 (t, 1H), 7.36 (m, 1H), 7.48 (dd, 1H), 7.87 (dd, 1H), 8.05 (d, 1H), 10.20 (s, 1H), 11.73 (s, 1H). |

### Beispiel 25

### 3-Amino-N-[3-fluor-4-(1H-pyrrolo[2,3-b]pyridin-4-yloxy)phenyl]cyclopentancarboxamid-Trifluoracetat

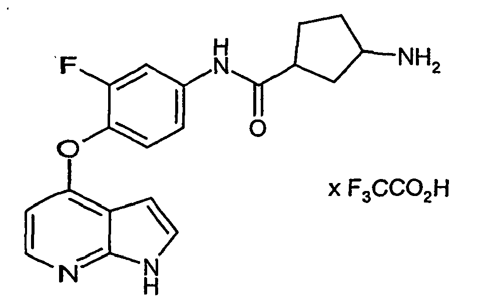

Zu einer Lösung aus 29 mg (0.065 mmol) tert-Butyl-[3-({[3-fluor-4-(1H-pyrrolo[2,3-b]pyridin-4-yloxy)phenyl]amino}carbonyl)cyclopentyl]carbamat (aus Beispiel 21A) in 1 ml Dichlormethan gibt man unter Argon 1 ml Trifluoressigsäure und lässt 2 Stunden bei Raumtemperatur rühren. Man entfernt das Lösungsmittel im Vakuum und reinigt den Rückstand durch präparative HPLC.

Ausbeute: 21.4 mg (68% d. Th.)
LC-MS (Methode 1): Rₜ = 1.03 min.
MS (ESI pos.): m/z = 355 (M+H)⁺

### Beispiel 26

### N-[3-Fluor-4-(1H-pyrrolo[2,3-b]pyridin-4-yloxy)phenyl]piperidin-2-carboxamid

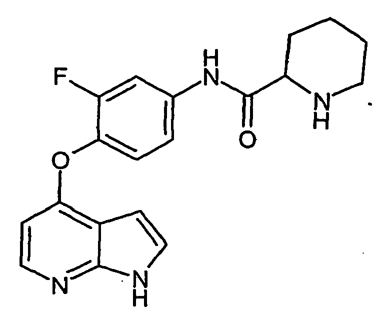

Zu einer Lösung aus 65 mg (0.144 mmol) tert-Butyl-2-({[3-fluor-4-(1H-pyrrolo[2,3-b]pyridin-4-yloxy)phenyl]amino}carbonyl)piperidin-1-carboxylat (aus Beispiel 23A) in 1 ml Dichlormethan gibt man unter Argon 1 ml Trifluoressigsäure und lässt 2 Stunden bei Raumtemperatur rühren. Man entfernt das Lösungsmittel im Vakuum. Der Rückstand wird in Dichlormethan gelöst und mit einer gesättigten Lösung von Natriumcarbonat gewaschen. Die organische Phase wird getrocknet und eingeengt. Man reinigt den Rückstand durch präparative HPLC.

Ausbeute: 37 mg (66% d. Th.)
LC-MS (Methode 1): Rₜ = 1.02 min.
MS (ESI pos.): m/z = 355 (M+H)⁺

### Beispiel 27

### 3-Amino-N-[3-fluor-4-(1H-pyrrolo[2,3-b]pyridin-4-yloxy)phenyl]cyclopentancarboxamid-Hydrochlorid

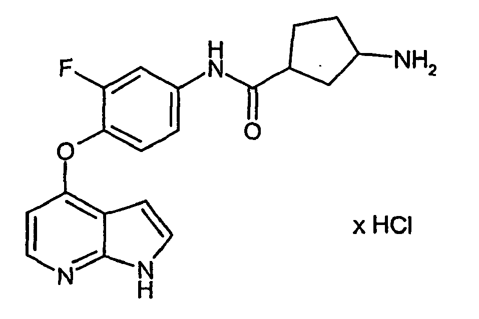

Zu einer Lösung aus 454 mg (1.00 mmol) tert-Butyl-[3-({[3-fluor-4-(1H-pyrrolo[2,3-b]pyridin4-yloxy)phenyl]amino}carbonyl)cyclopentyl]carbamat (aus Beispiel 21 A) in 5 ml Dioxan gibt man unter Argon bei 0°C 2.5 ml (10 mmol) einer 4M Lösung von Chlorwasserstoff in Dioxan und lässt über Nacht bei Raumtemperatur rühren. Man entfernt das Lösungsmittel im Vakuum und reinigt den Rückstand durch präparative HPLC.

Ausbeute: 271 mg (70% d. Th.)
LC-MS (Methode 1): Rₜ = 1.03 min.
MS (ESI pos.): m/z = 355 (M+H)⁺

¹H-NMR (DMSO-d₆, 300 MHz): δ = 1.72 (m, 1H), 1.80-2.08 (m, 4H), 2.28 (m, 1H), 3.00 (m, 1H), 6.40 (m, 1H), 6.61 (d, 1H), 7.43 (t, 1H), 7.51 (m, 2H), 7.93 (dd, 1H), 8.10 (br. s, 3H), 8.24 (d, 1H), 10.66 (s, 1H), 12.47 (s, 1H).

Analog zu Beispiel 27 werden hergestellt:

| **Bsp.-Nr.** | **Edukt (Bsp.- Nr.)** | **Struktur** | **MS, HPLC, LC-MS, ¹H-NMR** |
|---|---|---|---|
| **28** | 22A | 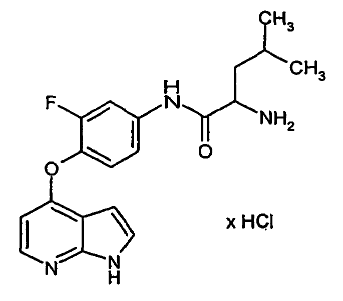 | LC-MS (Methode 1): Rₜ = 1.24 min. MS (ESI pos.): m/z = 357 (M+H)⁺ |
| **29** | 23A | 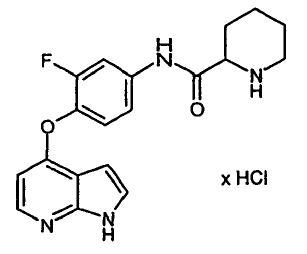 | LC-MS (Methode 1): Rₜ = 1.03 min. MS (ESI pos.): m/z = 355 (M+H)⁺ ¹H-NMR (DMSO-d₆, 300 MHz): δ = 1.45-1.89 (m, 5H), 2.31 (m, 1H), 2.97 (m, 1H), 3.29 (m, 1H), 6.40 (m, 1H), 6.61 (d, 1H), 7.48 (t, 1H), 7.51 (m, 1H), 7.56 (dd, 1H), 7.88 (dd, 1H), 8.22 (d, 1H), 8.85 (m, 1H), 9.40 (m, 1H), 11.36 (s, 1H), 12.38 (s, 1H). |
| **30** | 24A | 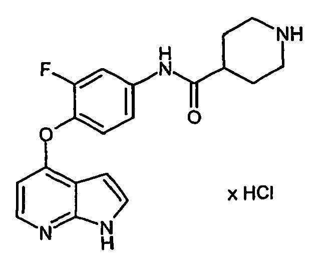 | LC-MS (Methode 1): Rₜ = 0.99 min. MS (ESI pos.): m/z = 355 (M+H)⁺ ¹H-NMR (DMSO-d₆, 200 MHz): δ = 1.71- 2.09 (m, 4H), 2.31 (m, 1H), 2.72 (m, 1H), 2.92 (m, 2H), 3.34 (m, 2H), 6.44 (m, 1H), 6.62 (d, 1H), 7.44 (t, 1H), 7.51 (m, 2H), 7.92 (dd, 1H), 8.25 (d, 1H), 8.72 (m, 1H), 9.06 (m, 1H), 10.70 (s, 1H), 12.54 (s, 1H). |
| **31** | 25A | 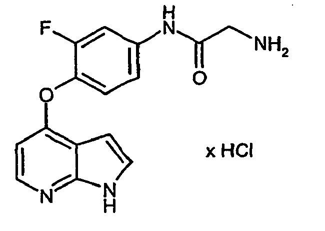 | LC-MS (Methode 5): Rₜ = 2.26 min. MS (ESI pos.): m/z = 301 (M+H)⁺ |
| **32** | 26A | 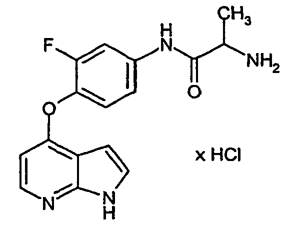 | LC-MS (Methode 1): Rₜ = 0.87 min. MS (ESI pos.): m/z = 315 (M+H)⁺ ¹H-NMR (DMSO-d₆, 300 MHz): δ = 1.51 (d, 3H), 4.14 (m, 1H), 6.43 (m, 1H), 6.67 (d, 1H), 7.51 (t, 1H), 7.54 (m, 1H), 7.59 (dd, 1H), 7.91 (dd, 1H), 8.26 (d, 1H), 8.41 (d, 3H), 11.42 (s, 1H), 12.57 (s, 1H). |
| **33** | 27A | 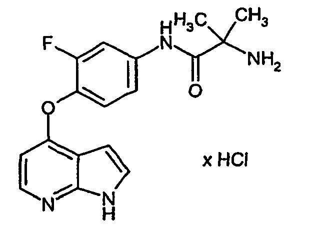 | LC-MS (Methode 6): Rₜ = 1.23 min. MS (ESI pos.): m/z = 329 (M+H)⁺ ¹H-NMR (DMSO-d₆, 300 MHz): δ = 1.68 (s, 6H), 6.41 (m, 1H), 6.62 (d, 1H), 7.49 (t, 1H), 7.52 (m, 1H), 7.71 (dd, 1H), 7.97 (dd, 1H), 8.23 (d, 1H), 8.49 (s, 3H), 10.74 (s, 1H), 12.43 (s, 1H). |
| **34** | 28A | 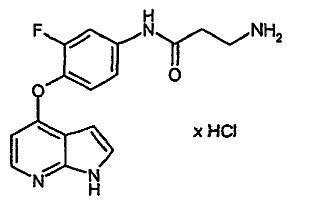 | LC-MS (Methode 5): Rₜ = 2.38 min. MS (ESI pos.): m/z = 315 (M+H)⁺ ¹H-NMR (DMSO-d₆, 300 MHz): δ = 2.42 (t, 2H), 2.86 (t, 2H), 6.22 (d, 1H), 6.35 (d, 1H), 7.32 (t, 1H), 7.35 (m, 1H), 7.37 (dd, 1H), 7.83 (dd, 1H), 8.06 (d, 1H), 8.49 (s, 3H), 11.73 (s, 1H). |
| **35** | 30A | 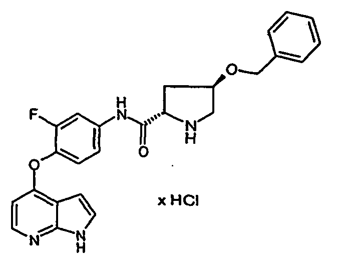 | LC-MS (Methode 6): Pₜ = 1.69 min. MS (ESI pos.): m/z = 447 (M+H)⁺ ¹H-NMR (DMSO-d₆, 200 MHz): δ = 2.09 (m, 1H), 2.77 (m, 1H), 3.48 (m, 2H), 4.39 (m, 1H), 4.57 (m, 3H), 6.35 (d, 1H), 6.55 (d, 1H), 7.25-7.45 (m, 5H), 7.49 (m, 3H), 7.87 (dd, 1H), 8.20 (d, 1H), 8.93 (m, 1H), 10.09 (m, 1H), 11.37 (s, 1H), 12.26 (s, 1H). |
| **36** | 31A | 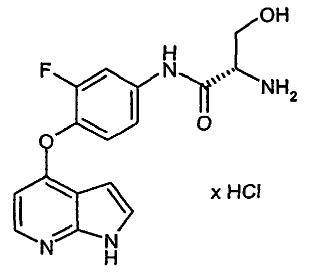 | LC-MS (Methode 6): Rₜ = 1.13 min. MS (ESI pos.): m/z = 331 (M+H)⁺ ¹H-NMR (DMSO-d₆, 300 MHz): δ = 4.11 (m, 2H), 6.41 (m, 1H), 6.63 (d, 1H), 7.49 (t, 1H), 7.52 (m, 1H), 7.57 (dd, 1H), 7.90 (dd, 1H), 8.24 (d, 1H), 8.37 (d, 3H), 11.33 (s, 1H), 12.48 (s, 1H). |

### Beispiel 37

### 2-Amino-N-[3-fluor-4-(1H-pyrrolo[2,3-b]pyridin-4-yloxy)phenyl]benzamid

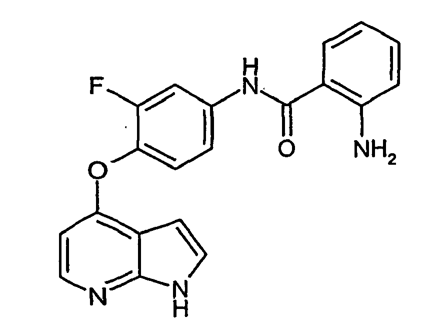

Zu einer Lösung aus 50 mg (0.12 mmol) N-[3-Fluor-4-(1H-pyrrolo[2,3-b]pyridin-4-yloxy)phenyl] 2-nitrobenzamid (aus Beispiel 29A) in 5 ml Ethanol gibt man Platin(IV)oxid und lässt über Nacht bei Raumtemperatur unter einer Wasserstoffatmosphäre rühren. Man filtriert vom Feststoff ab und entfernt das Lösungsmittel im Vakuum. Man reinigt durch präparative HPLC.

Ausbeute: 24 mg (50% d. Th.)
LC-MS (Methode 1): Rₜ = 1.86 min.
MS (ESI pos.): m/z = 363 (M+H)⁺

¹H-NMR (DMSO-d₆, 200 MHz): δ = 6.26 (m, 1H), 6.37 (m, 3H), 6.61 (t, 1H), 6.67 (d, 1H), 7.22 (t, 1H), 7.37 (m, 2H), 7.62 (t, 2H), 7.92 (dd, 1H), 8.07 (d, 1H), 10.26 (s, 1H), 11.77 (s, 1H).

### Beispiel 38

### (4R)-N-[3-Fluor-4-(1H-pyrrolo[2,3-b]pyridin-4-yloxy)phenyl]-4-hydroxy-L-prolinamid-Hydrochlorid

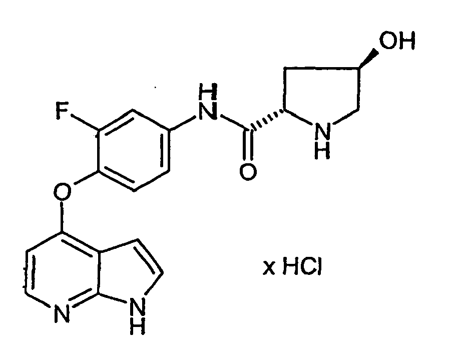

Zu einer Lösung aus 114 mg (0.23 mmol) (4R)-4-(Benzyloxy)-N-[3-fluor-4-(1H-pyrrolo[2,3-b]-pyridin-4-yloxy)phenyl]-L-prolinamid-Hydrochlorid (aus Beispiel 35) in 5 ml Ethanol gibt man 10 mg 10%iges Palladium auf Kohle und lässt über Nacht bei Raumtemperatur unter einer Wasserstoffatmosphäre rühren. Man filtriert vom Feststoff ab und entfernt das Lösungsmittel im Vakuum. Man reinigt durch präparative HPLC.

Ausbeute: 14 mg (15% d. Th.)
LC-MS (Methode 5): Rₜ = 1.16 min.
MS (ESI pos.): m/z = 357 (M+H)⁺

¹H-NMR (DMSO-d₆, 300 MHz): δ = 1.80 (m, 1H), 2.02 (dd, 1H), 2.80 (m, 1H), 2.92 (dd, 2H), 3.89 (t, 1H), 4.22 (s, 1H), 4.70 (d, 1H), 6.21 (d, 1H), 6.35 (d, 1H), 7.32 (t, 1H), 7.35 (d, 1H), 7.54 (dd, 1H), 7.90 (dd, 1H), 8.06 (d, 1H), 10.19 (m, 1H), 11.73 (s, 1H).

### Beispiel 39

### N-[3-Fluor-4-(1H-pyrrolo[2,3-b]pyridin-4-yloxy)phenyl]-4-methylpiperazin-1-carboxamid

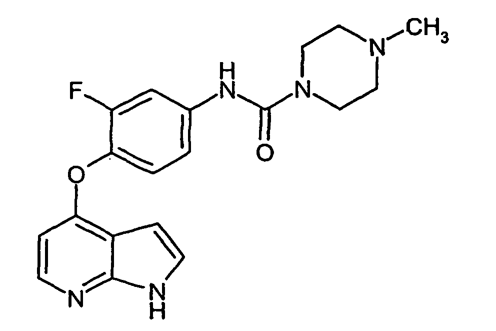

Zu einer Lösung aus 164 mg (61%ig, 0.27 mmol) Phenyl-[3-fluor-4-(1H-pyrrolo[2,3-b]pyridin-4-yloxy)phenyl]carbamat (aus Beispiel 20A) in 2 ml DMF gibt man 31 µl (0.27 mmol) 1-Methylpiperazin und lässt 3 Stunden bei 60°C rühren. Man reinigt die Lösung durch präparative HPLC.

Ausbeute: 57 mg (55% d. Th.)
LC-MS (Methode 5): Rₜ = 2.44 min.
MS (ESI pos.): m/z = 370 (M+H)⁺

¹H-NMR (DMSO-d₆, 300 MHz): δ = 2.78 (s, 3H), 3.05 (m, 2H), 3.30 (t, 2H), 3.43 (d, 2H), 4.31 (d, 2H), 6.41 (d, 1H), 6.62 (d, 1H), 7.38 (t, 1H), 7.44 (dd, 1H), 7.53 (m, 1H), 7.75 (dd, 1H), 8.26 (d, 1H), 9.33 (s, 1H), 10.94 (s, 1H), 12.51 (s, 1H).

### Beispiel 40

### N-[2-(Dimethylamino)ethyl]-N'-[3-fluor-4-(1H-pyrrolo[2,3-b]pyridin-4-yloxy)phenyl]harnstoff

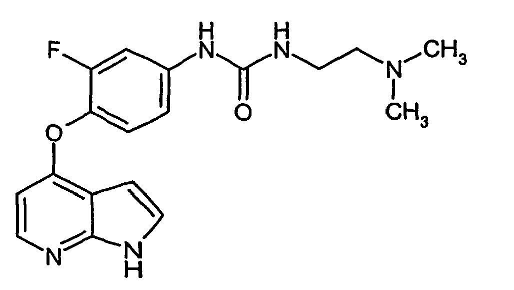

Zu einer Lösung aus 300 mg (44%ig, 0.367 mmol) Phenyl-[3-fluor-4-(1H-pyrrolo[2,3-b]pyridin-4-yloxy)phenyl]carbamat (aus Beispiel 20A) in 2 ml DMF gibt man 38 µl (0.367 mmol) N,N-Dimethylethylendiamin und lässt 3 Stunden bei 60°C rühren. Man reinigt die Lösung durch präparative HPLC.

Ausbeute: 122 mg (88% d. Th.)
LC-MS (Methode 1): Rₜ = 0.92 min.
MS (ESI pos.): m/z = 358 (M+H)⁺

¹H-NMR (DMSO-d₆, 300 MHz): δ = 2.17 (s, 6H), 2.33 (t, 2H), 3.19 (m, 2H), 6.15 (t, 1H), 6.22 (dd, 1H), 6.33 (d, 1H), 7.08 (m, 1H), 7.24 (t, 1H), 7.34 (dd, 1H), 7.53 (m, 1H), 7.65 (dd, 1H), 8.05 (d, 1H), 8.94 (s, 1H), 11.70 (s, 1H).

### Beispiel 41

### 2-(Dimethylamino)ethyl-[3-fluor-4-(1H-pyrrolo[2,3-b]pyridin-4-yloxy)phenyl]carbamat

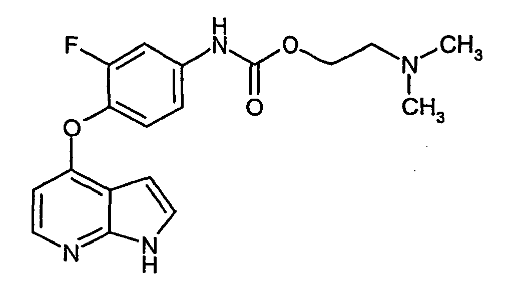

Zu einer Lösung aus 29 µl (0.29 mmol) N,N-Dimethylethanolamin in 2 ml wasserfreiem THF gibt man 0.58 ml (0.29 mmol) Kaliumhexamethyldisilazid (0.5 M in THF). Dazu tropft man eine Lösung aus 100 mg (88%ig, 0.24 mmol) Phenyl-[3-fluor-4-(1H-pyrrolo[2,3-b]pyridin-4-yloxy)-phenyl]carbamat (aus Beispiel 20A) in 2 ml wasserfreiem THF zu und lässt über Nacht bei RT rühren. Man setzt Wasser hinzu, extrahiert dreimal mit Essigsäureethylester, trocknet über Magnesiumsulfat und entfernt das Lösungsmittel im Vakuum. Der Rückstand wird durch präparative HPLC gereinigt.

Ausbeute: 19 mg (20% d. Th.)
LC-MS (Methode 1): Rₜ = 1.02 min.
MS (ESI pos.): m/z = 359 (M+H)⁺

¹H-NMR (DMSO-d₆, 300 MHz): δ = 2.20 (s, 6H), 2.53 (t, 2H), 4.19 (m, 2H), 6.22 (dd, 1H), 6.34 (d, 1H), 7.31 (m, 2H), 7.35 (dd, 1H), 7.53 (m, 1H), 7.59 (dd, 1H), 8.05 (d, 1H), 9.98 (s, 1H), 11.71 (s, 1H).

### Beispiel 42

### Pyrrolidin--3-yl-[3-fluor-4-(1H-pyrrolo[2,3-b]pyridin-4-yloxy)phenyl]carbamat

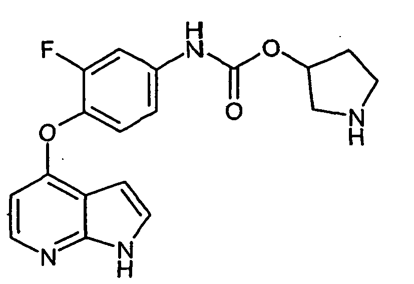

Analog Beispiel 26 wird die Titelverbindung aus 36 mg (0.080 mmol) tert-Butyl-3-[({[3-fluor-4-(1H-pyrrolo[2,3-b]pyridin-4-yloxy)phenyl]amino}carbonyl)oxy]pyrrolidin-1-carboxylat (aus Beispiel 32A) synthetisiert.

Ausbeute: 6.7 mg (33% d. Th.)
LC-MS (Methode 1): Rₜ = 0.95 min.
MS (ESI pos.): m/z = 357 (M+H)⁺

### Beispiel 43

### Isobutyl-{3-fluor-4-[(3-methyl-1H-pyrrolo[2,3-b]pyridin-4-yl)oxy]phenyl}carbamat

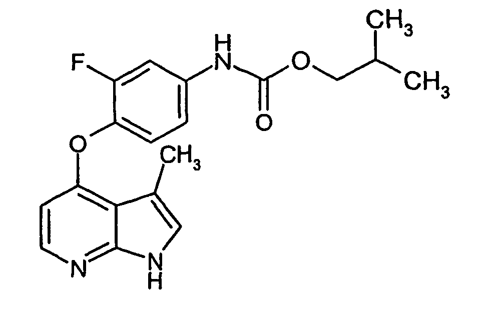

200 mg (0.77 mmol) {3-Fluor-4-[(3-methyl-1H-pyrrolo[2,3-b]pyridin-4-yl)oxy]phenyl}amin (aus Beispiel 15A) werden in 5 ml Dichlormethan suspendiert und auf 0°C gekühlt. Man tropft 110 µl (0.77 mmol) Triethylamin und 1.08 ml (1.16 mmol) Isobutylchlorformiat hinzu und lässt noch 2 Stunden bei 0°C rühren. Man gibt gesättigte Natriumhydrogencarbonatlösung hinzu und lässt 10 min bei RT rühren. Dann wird zweimal mit Dichlormethan extrahiert und die organische Lösung wird mit gesättigter Natriumchloridlösung gewaschen. Die vereinigten organischen Phasen werden über Magnesiumsulfat getrocknet und das Lösungsmittel im Vakuum entfernt. Das Rohprodukt wird in 8 ml Methanol aufgenommen. Man gibt 0.8 ml (0.8 mmol) 1 M Natriummethanolatlösung hinzu und lässt 30 min bei RT rühren. Man engt ein und reinigt durch präparative HPLC.

Ausbeute: 200 mg (72% d. Th.)
LC-MS (Methode 2): Rₜ = 2.41 min.
MS (ESI pos.): m/z = 358 (M+H)⁺.

¹H-NMR (DMSO-d₆, 300 MHz): δ = 0.94 (d, 6H), 1.94 (m, 1H), 3.90 (d, 2H), 6.13 (d, 1H), 7.12 (s, 1H), 7.29 (m, 2H), 7.60 (dd, 1H), 7.97 (d, 1H), 9.91 (s, 1H), 11.34 (br. s, 1H).

### Beispiel 44

### 2,2-Dimethylpropyl [3-fluor-4-(1H-pyrrolo[2,3-b]pyridin-4-yloxy)phenyl]carbamat

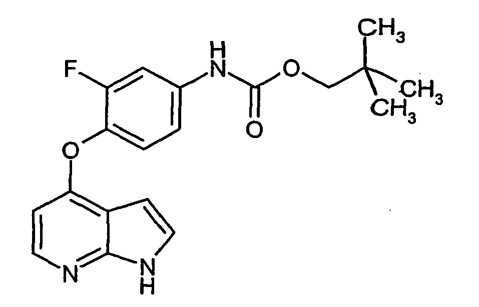

100 mg (0.41 mmol) [3-Fluor-4-(1H-pyrrolo[2,3-b]pyridin-4-yloxy)phenyl]amin (aus Beispiel 5A) und 660µl (6.62 mmol) Pyridin werden in Dichlormethan (5 ml) gelöst. Man kühlt auf 0°C, setzt 75 mg (0.49 mmol) Neopentylchlorformiat hinzu und lässt 24 h bei RT rühren. Dann gibt man Wasser zu und verdünnt mit Dichlormethan, extrahiert und trocknet die organische Phase über Natriumsulfat und entfernt das Lösungsmittel im Vakuum. Der Rückstand wird in 2.5 ml Methanol suspendiert und nach Zugabe von 0.09 ml (0.50 mmol) 5.4-molarer Natriummethanolatlösung 1 h bei RT gerührt. Man reinigt durch präparative HPLC und erhält einen Feststoff.

Ausbeute: 53 mg (43% d. Th.)
LC-MS (Methode 2): Rₜ = 2.45 min.
MS (ESI pos.): m/z = 358 (M+H)⁺

¹H-NMR (DMSO-d₆, 300 MHz): δ = 0.95 (s, 9H), 3.83 (s, 2H), 6.24 (dd, 1H), 6.33 (d, 1H), 7.35 (m, 3H), 7.62 (dd, 1H), 8.06 (dd, 1H), 9.94 (s, 1H), 11.76 (s, 1H).

Analog zu den aufgeführten Beispielen werden hergestellt:

| **Bsp.- Nr.** | **Struktur** | **LC-MS** |
|---|---|---|
| **45** | 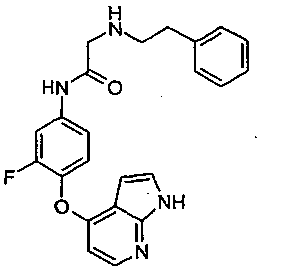 | LC-MS (Methode 2): Rₜ = 1.40 min. MS (ESI pos.): m/z = 405 (M+H)⁺ |
| **46** | 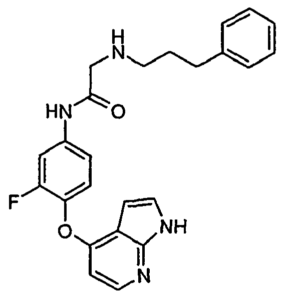 | LC-MS (Methode 2): Rₜ = 1.50 min. MS (ESI pos.): m/z = 419 (M+H)⁺ |
| **47** | 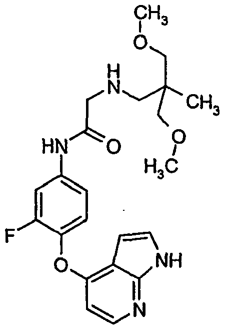 | LC-MS (Methode 2): Rₜ = 1.40 min. MS (ESI pos.): m/z = 431 (M+H)⁺ |
| **48** | 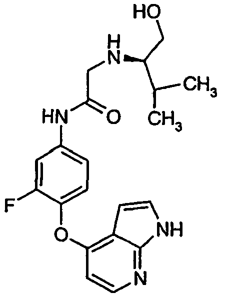 | LC-MS (Methode 2): Rₜ = 1.20 min. MS (ESI pos.): m/z = 387 (M+H)⁺ |
| **49** | 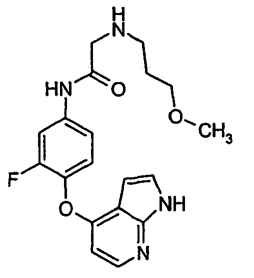 | LC-MS (Methode 2): Rₜ = 1.10 min. MS (ESI pos.): m/z = 373 (M+H)⁺ |
| **50** | 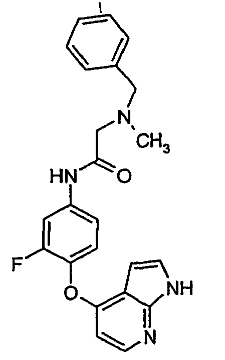 | LC-MS (Methode 2): Rₜ = 1.40 min. MS (ESI pos.): m/z = 405 (M+H)⁺ |
| **51** | 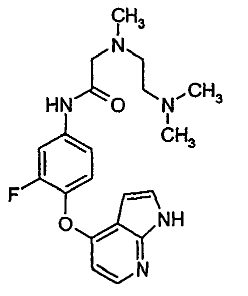 | LC-MS (Methode 2): Rₜ = 1.00 min. MS (ESI pos.): m/z = 386 (M+H)⁺ |
| **52** | 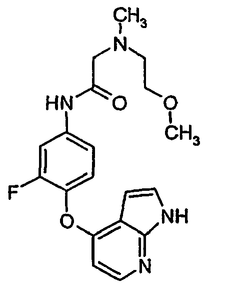 | LC-MS (Methode 2): Rₜ = 1.10 min. MS (ESI pos.): m/z = 373 (M+H)⁺ |

### Beispiel 53

### Isobutyl-{4-[(3-chlor-1H-pyrrolo[2,3-b]pyridin-4-yl)oxy]-3,5-difluorphenyl}carbamat

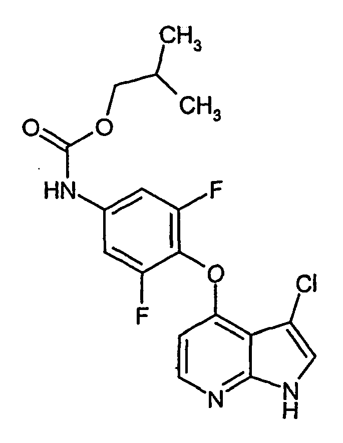

200 mg (0.50 mmol) 4-[(3-Chlor-1H-pyrrolo[2,3-b]pyridin-4-yl)oxy]-3,5-difluoranilin (aus Beispiel 37A) werden in 5 ml Dichlormethan suspendiert und auf 0°C gekühlt. Man tropft 70 µl (0.50 mmol) Triethylamin und 0.71 ml (0.75 mmol) Isobutylchlorformiat hinzu und lässt noch 2 Stunden bei 0°C rühren. Man gibt gesättigte Natriumhydrogencarbonatlösung hinzu und lässt 10 min bei RT rühren. Dann wird zweimal mit Dichlormethan extrahiert und die organische Lösung wird mit gesättigter Natriumchloridlösung gewaschen. Die vereinigten organischen Phasen werden über Magnesiumsulfat getrocknet und das Lösungsmittel im Vakuum entfernt. Das Rohprodukt wird in 6 ml Methanol aufgenommen. Man gibt 0.6 ml (0.6 mmol) 1 M Natriummethanolatlösung hinzu und lässt 30 min bei RT rühren. Man engt ein und reinigt durch präparative HPLC.

Ausbeute: 174 mg (65% d. Th.)
LC-MS (Methode 3): Rₜ = 2.82 min.
MS (ESI pos.): m/z = 396, 398 (M+H)⁺.

¹H-NMR (DMSO-d₆, 300 MHz): δ = 0.94 (d, 6H), 1.94 (m, 1H), 3.92 (d, 2H), 6.34 (d, 1H), 7.42 (d, 2H), 7.61 (d, 1H), 8.11 (d, 1H), 10.18 (s, 1H), 12.13 (br. s, 1H).

### B. Bewertung der physiologischen Wirksamkeit

In einem in vitro-Assay mit rekombinanter Rho-Kinase-II wird die Hemmung des Enzyms untersucht. Die gefäßrelaxierende Wirkung wird an Phenylephrin-induzierten Kontraktionen isolierter Ringe der Kaninchen-Arteria-Saphena bestimmt. Die Eignung der erfindungsgemäßen Verbindungen zur Behandlung von kardiovaskulären Erkrankungen kann durch Untersuchung der blutdrucksenkenden Wirkung an narkotisierten Ratten gezeigt werden.

### Hemmung der rekombinanten Rho-Kinase II (ROKα)

Die Aktivität der Rho-Kinase wird durch den Einbau von ³³P-Phosphat in ein Substratpeptid bestimmt. Dazu wird kommerziell erhältliche Rho-Kinase II (Upstate Biotechnology) in Gegenwart des S6 Phosphate-Acceptor-Peptides mit den Testsubstanzen oder einer Lösungsmittelkontrolle 10 min bei 37°C vorinkubiert. Anschließend wird die Kinase-Reaktion durch Zugabe von ³³P-markiertem ATP gestartet. Nach 20 min bei 37°C wird die Reaktion durch Zugabe von H₃PO₄ gestoppt. Aliquots werden auf Filter pipettiert, die Filter gewaschen und anschließend mit Scintillator beschichtet. Die Radioaktivität der am Filter gebundenen ³³P-markierten Peptide wird in einem Micro-Beta-Counter gemessen. Der IC₅₀-Wert entspricht der Konzentration einer Testsubstanz bei welcher der Rho-Kinase katalysierte Einbau von ³³P in das Peptid im Vergleich zu einer Lösungsmittelkontrolle um 50 % gehemmt ist. Die experimentellen Daten sind in der folgenden Tabelle zusammengestellt.

| **Beispiel-Nr.** | **IC₅₀ (nM)** |
|---|---|
| 15 | 49 |
| 23 | 12 |
| 40 | 27 |
| 41 | 20 |

### Gefäßrelaxierende Wirkung in vitro

3 mm breite Ringe der isolierten Kaninchen-Arteria-Saphena werden einzeln in 5 ml-Organbäder mit 37°C warmer, carbogenbegaster Krebs-Henseleit-Lösung gebracht. Der Gefäßtonus wird isometrisch erfasst und registriert. Kontraktionen werden durch Zugabe von 3x10⁻⁸ g/ml Phenylephrin induziert und nach 4 min wieder ausgewaschen. Nach mehreren Kontrollzyklen wird die zu untersuchende Substanz mit jedem weiteren Durchgang in steigender Dosierung vorinkubiert und die darauffolgende Kontraktion mit der Höhe der letzten Kontrollkontraktion verglichen. Es wird die Konzentration errechnet, die erforderlich ist, um die Höhe des Kontrollwertes um 50 % zu reduzieren (IC₅₀).

### Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäßer Verbindung wird der Suspension zugefügt. Unter Rühren erfolgt die Zugabe des Wassers. Bis zum Abschluss der Quellung des Rhodigels wird ca. 6h gerührt.

## Patentansprüche

1. Verbindung der Formel 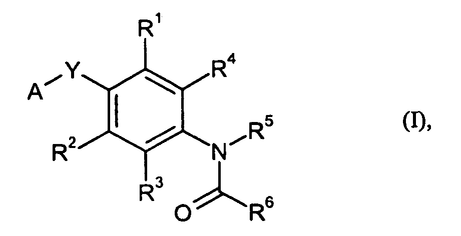 worin
A einen Rest 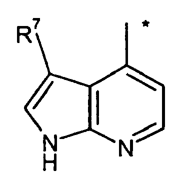 bedeutet,
worin
R⁷ für Wasserstoff, Chlor oder Methyl steht,
und
* für die Anknüpfstelle an Y steht,
Y O bedeutet,
R¹ und R² unabhängig voneinander Wasserstoff, Fluor oder Chlor bedeuten,
R³ und R⁴ unabhängig voneinander Wasserstoff oder Fluor bedeuten,
R⁵ Wasserstoff bedeutet,
R⁶ einen Rest bedeutet, der ausgewählt ist aus der Gruppe von:
(C₁-C₆)-Alkyl, das durch Amino, Hydroxy, (C₁-C₆)-Alkoxy, (C₁-C₆)-Alkylthio, (C₁-C₆)-Alkylamino, (C₅-C₆)-Cycloalkylamino, (C₁-C₆)-Alkylcarbonylamino, (C₁-C₆)-Alkoxycarbonyl, Phenyl, 5- oder 6-gliedriges Heteroaryl oder 5- oder 6-gliedriges Heterocyclyl substituiert ist,
worin Alkylamino, Cycloalkylamino oder Phenyl ihrerseits durch Hydroxy, Halogen, (C₁-C₃)-Alkoxy, (C₁-C₃)-Alkylamino oder Phenyl substituiert sein können,
(C₁-C₆)-Alkoxy, das durch Amino oder (C₁-C₆)-Alkylamino substituiert sein kann,
Cyclopentyl, Cyclohexyl, 5- oder 6-gliedrigem Heterocyclyl oder 5- oder 6-gliedrigem Heterocyclyloxy,
wobei Cyclopentyl, Cyclohexyl, Heterocyclyl oder Heterocyclyloxy durch Amino, Hydroxy, (C₁-C₃)-Alkyl, Oxo oder Benzyloxy substituiert sein können,
und Phenyl, Thienyl, Furyl, Pyrrolyl, Pyrazolyl, Thiazolyl, Oxazolyl, Imidazolyl, Pyridyl, Pyrimidyl oder Pyridazinyl,
wobei Phenyl, Thienyl, Furyl, Pyrrolyl, Pyrazolyl, Thiazolyl, Oxazolyl, Imidazolyl, Pyridyl, Pyrimidyl oder Pyridazinyl durch Amino, Hydroxy, Halogen, Cyano, (C₁-C₃)-Alkyl, das seinerseits durch Amino oder (C₁-C₆)-Alkylamino substituiert sein kann, (C₁-C₃)-Alkoxy oder (C₁-C₃)-Alkoxycarbonyl substituiert sein können,
und ihre Salze, Hydrate, Hydrate der Salze und Solvate.

2. Verbindung der Formel (I) nach Anspruch 1,
worin
A einen Rest 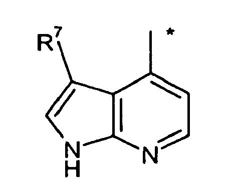 bedeutet,
worin
R⁷ für Wasserstoff, Chlor oder Methyl steht,
und
* für die Anknüpfstelle an Y steht,
Y O bedeutet,
R¹ und R² unabhängig voneinander Wasserstoff oder Fluor bedeuten,
R³ und R⁴ Wasserstoff bedeuten,
R⁵ Wasserstoff bedeutet,
R⁶ einen Rest bedeutet, der ausgewählt ist aus der Gruppe von:
(C₁-C₆)-Alkyl, das durch Amino, Hydroxy, (C₁-C₆)-Alkylamino, Cyclohexylamino oder Piperidinyl substituiert ist,
worin Alkylamino oder Cyclohexylamino ihrerseits durch Hydroxy oder Phenyl substituiert sein können,
(C₁-C₆)-Alkoxy, das durch Amino oder (C₁-C₆)-Alkylamino substituiert sein kann,
Cyclopentyl, Piperazinyl, Piperidinyl, Pyrrolidinyl, Piperidinyloxy oder Pyrrolidinyloxy,
wobei Cyclopentyl, Piperazinyl, Piperidinyl, Pyrrolidinyl, Piperidinyloxy oder Pyrrolidinyloxy durch Amino, Hydroxy, (C₁-C₃)-Alkyl oder Benzyloxy substituiert sein können,
und Phenyl oder Thienyl,
wobei Phenyl oder Thienyl durch (C₁-C₃)-Alkyl, das seinerseits durch Amino oder (C₁-C₆)-Alkylamino substituiert sein kann, substituiert sein können,
und ihre Salze, Hydrate, Hydrate der Salze und Solvate.

3. Verfahren zur Herstellung von Verbindungen der Formel (I), wie in Anspruch 1 definiert, **dadurch gekennzeichnet, dass** man entweder
**[A]** Verbindungen der Formel 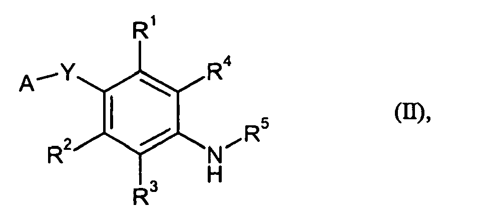 worin
A, Y, R¹, R², R³, R⁴ und R⁵ die in Anspruch 1 angegebene Bedeutung aufweisen,
mit Verbindungen der Formel 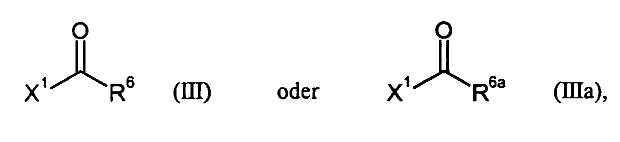 worin
R⁶ die in Anspruch 1 angegebene Bedeutung aufweist,
R^{6a} einem wie oben definierten Rest R⁶ entspricht, der aber anstelle einer sekundären oder tertiären Aminogruppe einen Chlorsubstituenten oder anstelle einer freien Aminogruppe eine Nitrogruppe oder eine geschützte Aminogruppe enthält und
X¹ für Halogen, bevorzugt Chlor oder Brom, oder Hydroxy steht,
umsetzt
und im Fall der Umsetzung mit Verbindungen (IIIa) anschließend noch im Rest R^{6a} den Chlorsubstituenten durch ein Amin substituiert, die Nitrogruppe zur entsprechenden Aminogruppe hydriert oder die Schutzgruppe zur Freisetzung der entsprechenden freien Aminogruppe abspaltet
oder
**[B]** Verbindungen der Formel 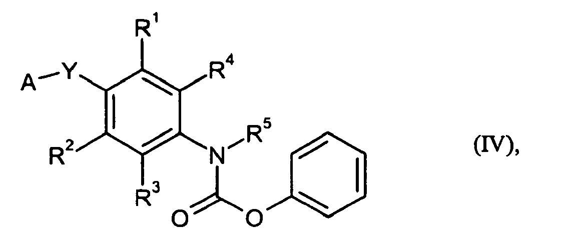 worin
A, Y, R¹, R², R³, R⁴ und R⁵ die in Anspruch 1 angegebene Bedeutung aufweisen,
mit Verbindungen der Formel
H₂N-R⁸ (V),
worin
R⁸ die in Anspruch 1 angegebene Bedeutung aufweist,
umsetzt.

4. Verbindung, wie in einem der Ansprüche 1 oder 2 definiert, zur Behandlung und/oder Prophylaxe von Erkrankungen.

5. Verwendung einer Verbindung, wie in einem der Ansprüche 1 oder 2 definiert, zur Herstellung von Arzneimitteln zur Behandlung und/oder Prophylaxe von kardiovaskulären Erkrankungen.

6. Verwendung einer Verbindung, wie in einem der Ansprüche 1 oder 2 definiert, zur Herstellung von Arzneimitteln zur Behandlung und/oder Prophylaxe von erektiler Dysfunktion.

7. Arzneimittel enthaltend eine Verbindung, wie in einem der Ansprüche 1 oder 2 definiert, in Kombination mit einem weiteren Wirkstoff.

8. Arzneimittel enthaltend eine Verbindung, wie in einem der Ansprüche 1 oder 2 definiert, in Kombination mit einem inerten nichttoxischen, pharmazeutisch geeigneten Hilfsstoff.

9. Arzneimittel nach Anspruch 7 oder 8 zur Behandlung und/oder Prophylaxe von kardiovaskulären Erkrankungen.

10. Arzneimittel nach Anspruch 7 oder 8 zur Behandlung und/oder Prophylaxe von erektiler Dysfunktion.

## Claims

1. Compound of the formula 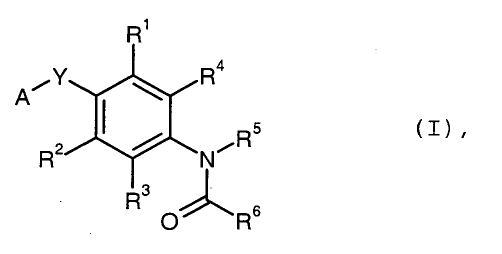 in which
A represents a radical 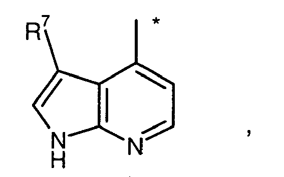
in which
R⁷ represents hydrogen, chlorine or methyl,
and
* represents the point of attachment to Y,
Y represents O,
R¹ and R² independently of one another represent hydrogen, fluorine or chlorine,
R³ and R⁴ independently of one another represent hydrogen or fluorine,
R⁵ represents hydrogen,
R⁶ represents a radical selected from the group consisting of:
(C₁-C₆)-alkyl which is substituted by amino, hydroxyl, (C₁-C₆)-alkoxy, (C₁-C₆)-alkylthio, (C₁-C₆)-alkylamino, (C₅-C₆)-cycloalkylamino, (C₁-C₆)-alkylcarbonylamino, (C₁-C₆)-alkoxycarbonyl, phenyl, 5- or 6-membered heteroaryl or 5- or 6-membered heterocyclyl,
where alkylamino, cycloalkylamino or phenyl for their part may be substituted by hydroxyl, halogen, (C₁-C₃)-alkoxy, (C₁-C₃)-alkylamino or phenyl,
(C₁-C₆)-alkoxy which may be substituted by amino or (C₁-C₆)-alkylamino,
cyclopentyl, cyclohexyl, 5- or 6-membered heterocyclyl or 5- or 6-membered heterocyclyloxy,
where cyclopentyl, cyclohexyl, heterocyclyl or heterocyclyloxy may be substituted by amino, hydroxyl, (C₁-C₃)-alkyl, oxo or benzyloxy,
and phenyl, thienyl, furyl, pyrrolyl, pyrazolyl, thiazolyl, oxazolyl, imidazolyl, pyridyl, pyrimidyl or pyridazinyl,
where phenyl, thienyl, furyl, pyrrolyl, pyrazolyl, thiazolyl, oxazolyl, imidazolyl, pyridyl, pyrimidyl or pyridazinyl may be substituted by amino, hydroxyl, halogen, cyano, (C₁-C₃)-alkyl, which for its part may be substituted by amino or (C₁-C₆)-alkylamino, (C₁-C₃)-alkoxy or (C₁-C₃)-alkoxycarbonyl,
and its salts, hydrates, hydrates of the salts and solvates.

2. Compound of the formula (I) according to Claim 1,
in which
A represents a radical 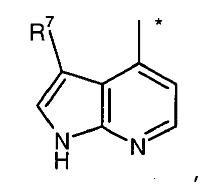
in which
R⁷ represents hydrogen, chlorine or methyl
and
* represents the point of attachment to Y,
Y represents O,
R¹ and R² independently of one another represent hydrogen or fluorine,
R³ and R⁴ represent hydrogen,
R⁵ represents hydrogen,
R⁶ represents a radical selected from the group consisting of:
(C₁-C₆)-alkyl which is substituted by amino, hydroxyl, (C₁-C₆)-alkylamino, cyclohexylamino or piperidinyl,
where alkylamino or cyclohexylamino for their part may be substituted by hydroxyl or phenyl,
(C₁-C₆)-alkoxy which may be substituted by amino or (C₁-C₆)-alkylamino,
cyclopentyl, piperazinyl, piperidinyl, pyrrolidinyl, piperidinyloxy or pyrrolidinyloxy,
where cyclopentyl, piperazinyl, piperidinyl, pyrrolidinyl, piperidinyloxy or pyrrolidinyloxy may be substituted by amino, hydroxyl, (C₁-C₃)-alkyl or benzyloxy,
and phenyl or thienyl,
where phenyl or thienyl may be substituted by (C₁-C₃)-alkyl which for its part may be substituted by amino or (C₁-C₆)-alkylamino,
and its salts, hydrates, hydrates of the salts and solvates.

3. Process for preparing compounds of the formula (I) as defined in Claim 1, **characterized in that** either
**[A]** compounds of the formula 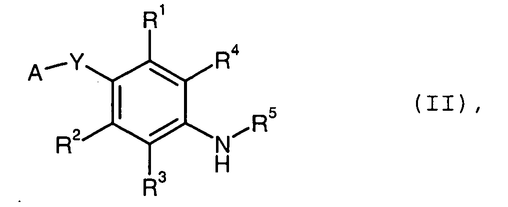 in which
A, Y, R¹, R², R³, R⁴ and R⁵ are as defined in Claim 1
are reacted with compounds of the formula 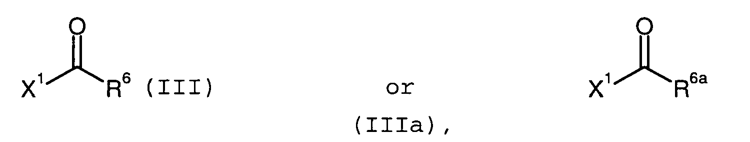 in which
R⁶ is as defined in Claim 1,
R^{6a} corresponds to a radical R⁶ as defined above which, however, contains, instead of a secondary or tertiary amino group, a chlorine substituent or, instead of a free amino group, a nitro group or a protected amino group, and
X¹ represents halogen, preferably chlorine or bromine, or hydroxyl,
and, in the case of the reaction with compounds (IIIa) in the radical R^{6a}, the chlorine substituent is subsequently substituted by an amine, the nitro group is hydrogenated to give the corresponding amino group or the protective group is cleaved off to release the corresponding free amino group
or
**[B]** compounds of the formula 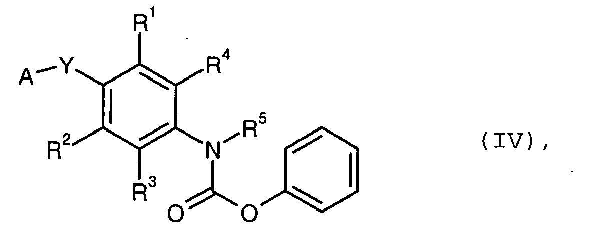 in which
A, Y, R¹, R², R³, R⁴ and R⁵ are as defined in Claim 1
are reacted with compounds of the formula
H₂N-R⁸ (V),
in which
R⁸ is as defined in Claim 1.

4. Compound as defined in either of Claims 1 or 2 for the treatment and/or prophylaxis of disorders.

5. Use of a compound as defined in either of Claims 1 or 2 for preparing medicaments for the treatment and/or prophylaxis of cardiovascular disorders.

6. Use of a compound as defined in either of Claims 1 or 2 for preparing medicaments for the treatment and/or prophylaxis of erectile dysfunction

7. Medicament comprising a compound as defined in either of Claims 1 or 2 in combination with a further active compound.

8. Medicament comprising a compound as defined in either of Claims 1 or 2 in combination with an inert non-toxic pharmaceutically suitable auxiliary.

9. Medicament according to Claim 7 or 8 for the treatment and/or prophylaxis of cardiovascular disorders.

10. Medicament according to Claim 7 or 8 for the treatment and/or prophylaxis of erectile dysfunction

## Revendications

1. Composé de formule 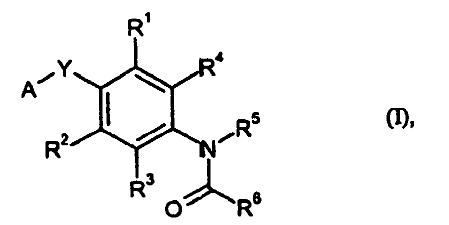 où
A représente un reste 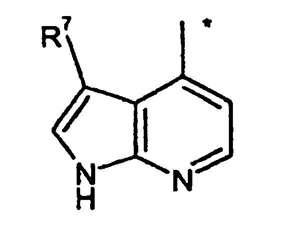 dans lequel
R⁷ représente l'hydrogène, le chlore ou un reste méthyle,
et
* désigne la position de rattachement à Y,
Y représente O,
R¹ et R² représentent, indépendamment l'un de l'autre, l'hydrogène, le fluor ou le chlore,
R³ et R⁴ représentent, indépendamment l'un de l'autre, l'hydrogène ou le fluor,
R⁵ représente l'hydrogène,
R⁶ représente un reste qui est choisi dans le groupe constitué de :
alkyle en C₁ à C₆, qui est substitué par un radical amino, hydroxy, alkoxy en C₁ à C₆, alkylthio en C₁ à C₆, alkylamino en C₁ à C₆, cycloalkylamino en C₅ ou C₆, (alkyle en C₁ à C₆)carbonylamino, (alkoxy en C₁ à C₆)-carbonyle, phényle, hétéroaryle pentagonal ou hexagonal, ou hétérocyclyle pentagonal ou hexagonal,
où les restes alkylamino, cycloalkylamino ou phényle peuvent eux-mêmes être substitués par un radical hydroxy, halogéno, alkoxy en C₁ à C₃, alkylamino en C₁ à C₃ ou phényle,
un reste alkoxy en C₁ à C₆ qui peut être substitué par un radical amino ou alkylamino en C₁ à C₆,
un reste cyclopentyle, cyclohexyle, hétérocyclyle pentagonal ou hexagonal ou hétérocyclyloxy pentagonal ou hexagonal,
les restes cyclopentyle, cyclohexyle, hétérocyclyle ou hétérocyclyloxy pouvant être substitués par un radical amino, hydroxy, alkyle en C₁ à C₃, oxo ou benzyloxy,
et un reste phényle, thiényle, furyle, pyrrolyle, pyrazolyle, thiazolyle, oxazolyle, imidazolyle, pyridyle, pyrimidyle ou pyridazinyle,
les restes phényle, thiényle, furyle, pyrrolyle, pyrazolyle, thiazolyle, oxazolyle, imidazolyle, pyridyle, pyrimidyle ou pyridazinyle pouvant être substitués par un radical amino, hydroxy, halogéno, cyano, alkyle en C₁ à C₃ qui peut lui-même être substitué par un radical amino ou alkylamino en C₁ à C₆, alkoxy en C₁ à C₃ ou (alkoxy en C₁ à C₃)-carbonyle,
et ses sels, ses hydrates, les hydrates des sels et les produits de solvatation.

2. Composé de formule (I) suivant la revendication 1,
où
A représente un reste 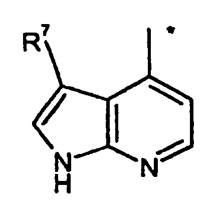 dans lequel
R⁷ représente l'hydrogène, le chlore ou un reste méthyle,
et
* désigne la position de rattachement à Y,
Y représente O,
R¹ et R² représentent, indépendamment l'un de l'autre, l'hydrogène ou le fluor,
R³ et R⁴ représentent l'hydrogène,
R⁵ représente l'hydrogène,
R⁶ représente un reste qui est choisi dans le groupe constitué de :
alkyle en C₁ à C₆, qui est substitué par un radical amino, hydroxy, alkylamino en C₁ à C₆, cyclohexylamino ou pipéridinyle,
où les restes alkylamino ou cyclohexylamino peuvent eux-mêmes être substitués par un radical hydroxy ou phényle,
un reste alkoxy en C₁ à C₆ qui peut être substitué par un radical amino ou alkylamino en C₁ à C₆,
un reste cyclopentyle, pipérazinyle, pipéridinyle, pyrrolidinyle, pipéridinyloxy ou pyrrolidinyloxy,
les restes cyclopentyle, pipérazinyle, pipéridinyle, pyrrolidinyle, pipéridinyloxy ou pyrrolidinyloxy pouvant être substitués par un radical amino, hydroxy, alkyle en C₁ à C₃ ou benzyloxy,
et un reste phényle ou thiényle,
les restes phényle ou thiényle pouvant être substitués par un radical alkyle en C₁ à C₃ qui peut lui-même être substitué par un radical amino ou alkylamino en C₁ à C₆,
et ses sels, ses hydrates, les hydrates des sels et les produits de solvatation.

3. Procédé de production de composés de formule (I) telle que définie dans la revendication 1, **caractérisé en ce que**
**[A]** on fait réagir des composés de formule 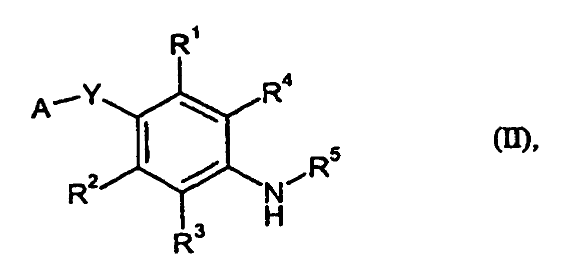 dans laquelle
A, Y, R¹, R², R³, R⁴ et R⁵ ont la définition indiquée dans la revendication 1,
avec des composés de formule 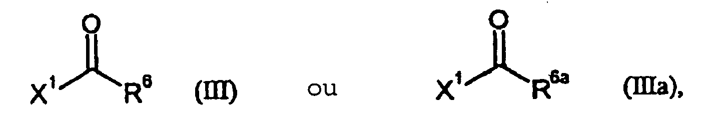 dans laquelle
R⁶ a la définition indiquée dans la revendication 1,
R^{6a} correspond à un reste R⁶ tel que défini ci-dessus mais qui contient, au lieu d'un groupe amino secondaire ou tertiaire, un substituant chloro, ou au lieu d'un groupe amino libre, un groupe nitro ou un groupe amino protégé, et
X¹ représente un halogène, avantageusement le chlore ou le brome, ou un groupe hydroxy,
puis, dans le cas de la réaction avec des composés (IIIa), on remplace encore dans le reste R^{6a} le substituant chloro par une amine, on hydrogène le groupe nitro en le groupe amino correspondant, ou on élimine le groupe protecteur pour libérer le groupe amino libre correspondant,
ou bien
**[B]** on fait réagir des composés de formule 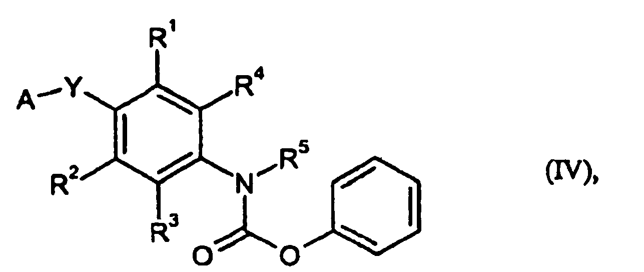
dans laquelle
A, Y, R¹, R², R³, R⁴ et R⁵ ont la définition indiquée dans la revendication 1,
avec des composés de formule
**H₂**N-R**⁸** **(V),**
dans laquelle
R⁸ a la définition indiquée dans la revendication 1.

4. Composé tel que défini dans l'une des revendications 1 et 2, destiné au traitement et/ou à la prophylaxie de maladies.

5. Utilisation d'un composé tel que défini dans l'une des revendications 1 et 2, pour la préparation de médicaments destinés au traitement et/ou à la prophylaxie de maladies cardio-vasculaires.

6. Utilisation d'un composé tel que défini dans l'une des revendications 1 et 2, pour la préparation de médicaments destinés au traitement et/ou à la prophylaxie d'un dysfonctionnement de l'érection.

7. Médicament contenant un composé tel que défini dans l'une des revendications 1 et 2, en combinaison avec une autre substance active.

8. Médicament contenant un composé tel que défini dans l'une des revendications 1 et 2, en combinaison avec une substance auxiliaire inerte non toxique pharmaceutiquement acceptable.

9. Médicament suivant la revendication 7 ou 8, destiné au traitement et/ou à la prophylaxie de maladies cardio-vasculaires.

10. Médicament suivant la revendication 7 ou 8, destiné au traitement et/ou à la prophylaxie du dysfonctionnement de l'érection.
